# EUROPEAN PATENT SPECIFICATION

(11) **EP 4 612 317 B1**
(45) Date of publication and mention of the grant of the patent: **09.09.2026**
(21) Application number: 23813245.0
(22) Date of filing: 01.11.2023
(51) Int. Cl.: C12Q 1/6804, C12Q 1/6841

(54) **SIMULTANEOUS IMAGING OF NUCLEIC ACID AND PROTEIN IN A SAMPLE**
SIMULTANE ABBILDUNG VON NUKLEINSÄURE UND PROTEIN IN EINER PROBE
IMAGERIE SIMULTANÉE D'ACIDES NUCLÉIQUES ET DE PROTÉINES DANS UN ÉCHANTILLON

(30) Priority: 01.11.2022 US 202263421390 P
(43) Date of publication of application: 10.09.2025
(73) Proprietor: GENENTECH, INC., South San Francisco, CA 94080 (US)
(72) Inventor: LUBECK, Eric, Scott, South San Francisco, CA 94080-4990 (US); MCGINNIS, Lisa, Michelle, South San Francisco, CA 94080-4990 (US); MONCADA, Reuben, South San Francisco, CA 94080-4990 (US); ROZENBLATT-ROSEN, Orit, South San Francisco, CA 94080-4990 (US)
(74) Representative: Küng, Peter
(86) International application number: PCT/US2023/078329
(87) International publication number: WO 2024/097740

(56) References cited:
- WO-A1-2012/152942
- WO-A1-2015/071445

## Description

### FIELD

The present disclosure relates to the simultaneous imaging of nucleic acids and proteins in a sample.

### BACKGROUND

Analyses of nucleic acid and protein abundances and distributions are useful in understanding complex biological systems. Conventional strategies for detecting and characterizing nucleic acids and proteins, *e.g., in situ* hybridization, Western blot analysis, and immunofluorescence detection, have been helpful in identifying target nucleic acids and proteins involved in overall organism growth and development as well as specifically interrogating the causes and progression of a wide variety of diseases. These strategies, however, do not allow for the simultaneous detection of nucleic acids and proteins in a single sample. For example, the fixation and permeabilization techniques commonly required for nucleic acid detection can prevent accurate protein detection in that same sample. Integrating multimodal analyses not only has the ability to enhance discovery and description of potential correlations between nucleic acid, *e.g.,* mRNA, abundances and distributions, and target protein abundances and distributions, but can also facilitate the identification of cell phenotypes. Given the advantages associated with multimodal analyses, there is a need in the art for additional methods allowing for the simultaneous imaging of nucleic acids and protein. WO 2012/152941 discloses a method for detecting analytes in a sample utilizes unfolding proximity probes to increase assay specificity and reduce background signal. Analytes can be DNA and proteins. Each probe contains an analyte-binding domain and a nucleic acid domain, with at least one probe featuring a hairpin structure that shields its reactive elements from premature interaction.

### SUMMARY

In a first aspect, the present disclosure provides a method for imaging a target protein and a target nucleic acid in a sample, comprising:
(a) using a provided a sample;
(b) contacting the sample with a protein binding reagent that specifically binds to a target protein in the sample, wherein the protein binding reagent is coupled to an oligonucleotide;
(c) contacting the sample with a blocking oligonucleotide comprising a nucleotide sequence that is complementary to a sequence of the oligonucleotide coupled to the protein binding reagent;
(d) amplifying a target nucleic acid in the sample to generate an amplicon by performing an amplification process;
(e) contacting the sample with a first labeled detection probe comprising a sequence that is complementary to a sequence of the oligonucleotide coupled to the protein binding reagent;
(f) imaging the first labeled detection probe to detect the target protein;
(g) contacting the sample with a second labeled detection probe comprising a sequence that is complementary to a sequence of the amplicon; and
(h) imaging the second labeled detection probe to detect the target nucleic acid.

In a second aspect, the present invention provides a method for imaging a target protein and a target nucleic acid in a sample, comprising:
(a) binding a protein binding reagent coupled to an oligonucleotide to a target protein in the sample;
(b) hybridizing a blocking oligonucleotide to the oligonucleotide coupled to the protein binding reagent;
(c) amplifying a target nucleic acid in the sample to generate an amplicon by performing an amplification process;
(d) imaging the target protein by detecting a first labeled detection probe that is hybridized to the oligonucleotide coupled to the protein binding reagent; and
(e) imaging the target nucleic acid by detecting a second labeled detection probe that is hybridized to the amplicon.

In certain embodiments, the amplification process is a rolling circle amplification process.

In certain embodiments, the rolling circle amplification process comprises:
(a) contacting the sample with (i) a padlock probe comprising two nucleotide sequences that are complementary to the target nucleic acid and (ii) a ligase to generate a circular DNA template; and
(b) performing a rolling circle amplification process to generate an amplicon from the circular DNA template.

In certain embodiments, the protein binding reagent is an antibody or an antigen binding fragment thereof.

In certain embodiments, the target nucleic acid comprises RNA.

In certain embodiments, the target nucleic acid is the oligonucleotide coupled to the protein binding reagent, the blocking oligonucleotide or a combination thereof.

In certain embodiments, the nucleotide sequence that is complementary to the oligonucleotide coupled to protein binding reagent is located at the 5' end of the blocking oligonucleotide.

In certain embodiments, the oligonucleotide coupled to protein binding reagent comprises a barcode sequence, and wherein the blocking oligonucleotide does not bind to the barcode sequence.

In certain embodiments, the blocking oligonucleotide comprises one or more modified nucleotides.

In certain embodiments, the 3' end of the blocking oligonucleotide comprises from about 1 to about 10 nucleotides with a phosphorothioate linkage.

In certain embodiments, providing a sample comprises one or more of the following:
(a) treating the sample with a fixative;
(b) dehydrating the sample; and
(c) permeabilizing the sample.

In certain embodiments, at least 10 target proteins and at least 10 target nucleic acids are imaged in the sample.

In certain embodiments, the sample is treated with NHS-acetate prior to amplifying the target nucleic acid.

In certain embodiments, the sample is a tissue sample.

### BRIEF DESCRIPTION OF THE FIGURES

**FIG. 1** provides a schematic of an exemplary method according to the present disclosure.
**FIG. 2** provides a schematic of an exemplary method according to the present disclosure, wherein the method comprises cyclic imaging.
**FIG. 3** provides images of a sample stained for both protein and mRNA using an exemplary method according to the present disclosure. The sample was stained for CD97B protein and Cd79a RNA to label B-cells and stained for F4/80 to label macrophages.
**FIG. 4** provides images illustrating the degradation of the 3' terminus of an oligonucleotide coupled to an antibody by Phi29 and the protection of the 3' terminus of the oligonucleotide using a blocking oligonucleotide (e.g., an exonuclease blocking oligonucleotide).
**FIG. 5** provides images of a sample stained for both protein and mRNA over multiple imaging cycles using an exemplary method according to the present disclosure. The sample was stained and imaged for CD22 protein and mRNA, then for CD4 protein and mRNA, and finally for F4/80 protein and mRNA.

### DETAILED DESCRIPTION

The present disclosure relates to compositions and methods for the simultaneous imaging of nucleic acids and protein in a single sample. For example, the methods of the present disclosure include the staining of one or more proteins followed by the staining of one or more nucleic acids, e.g., mRNAs, in a single sample, followed by imaging of both the proteins and nucleic acids.

For clarity, but not by way of limitation, the detailed description of the presently disclosed subject matter is divided into the following subsections:
I. Definitions;
II. Compositions & Methods for the Simultaneous Imaging of Nucleic Acids and Proteins; and
III. Systems and Kits.

### I. DEFINITIONS

Unless defined otherwise, all technical and scientific terms used herein have the meaning commonly understood by a person skilled in the art to which the subject matter of the present disclosure belongs. The following references provide one of skill with a general definition of many of the terms used in the present disclosure: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

As used herein, the use of the word "a" or "an" when used in conjunction with the term "comprising" in the claims and/or the specification can mean "one," but it is also consistent with the meaning of "one or more," "at least one," and "one or more than one."

The term "about" or "approximately" means within an acceptable error range for the particular value as determined by one of ordinary skill in the art, which depends in part on how the value is measured or determined, *i.e.,* the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

The term "amplification process" refers generally to any process where a portion of a nucleic acid is copied or replicated into at least one additional nucleic acid molecule.

The term "antibody" herein is used in the broadest sense and encompasses various antibody structures, including but not limited to monoclonal antibodies, polyclonal antibodies, multispecific antibodies (*e.g.,* bispecific antibodies) and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂, diabodies, linear antibodies, single-chain antibody molecules (*e.g.,* scFv) and multispecific antibodies formed from antibody fragments.

The terms "comprise(s)," "include(s)," "having," "has," "can," "contain(s)" and variants thereof, as used herein, are intended to be open-ended transitional phrases, terms or words that do not preclude additional acts or structures. The present disclosure also contemplates other embodiments "comprising," "consisting of" and "consisting essentially of," the embodiments or elements presented herein, whether explicitly set forth or not.

The term "coupled" can refer to the connecting or uniting of two or more components by an interaction, bond, link, force or tie in order to keep two or more components together. In certain embodiments, the term "coupled" encompasses either direct or indirect binding where, for example, a first component is directly bound to a second component, or one or more intermediate molecules are disposed between the first component and the second component. Exemplary bonds comprise covalent bonds, ionic bonds, van der Waals interactions and other bonds identifiable by a skilled person.

The terms "detect" or "detection," as used herein, indicate the determination of the existence and/or presence of a target, *e.g.,* a protein target or a nucleic acid target, in a limited portion of space, including but not limited to a sample. The terms "detect" or "detection," as used herein, can comprise determination of chemical and/or biological properties of the target, including but not limited to ability to interact, and in particular bind, other compounds, ability to activate another compound and additional properties identifiable by a skilled person upon reading of the present disclosure. The detection can be quantitative or qualitative. A detection is "quantitative" when it refers, relates to, or involves the measurement of quantity or amount of the target or signal (also referred as quantitation), which includes but is not limited to any analysis designed to determine the amounts or proportions of the target or signal. A detection is "qualitative" when it refers, relates to, or involves identification of a quality or kind of the target or signal in terms of relative abundance to another target or signal, which is not quantified.

As used herein, the term "hybridization," refers to the process in which two single-stranded polynucleotides bind non-covalently to form a stable double-stranded polynucleotide.

As used herein, the term "imaging" refers to microscopy. In certain embodiments, microscopy includes immunofluorescence microscopy.

As used herein, the term "individual" or "subject" refers to a vertebrate or an invertebrate, such as a human or non-human animal, for example, a mammal. Mammals include, but are not limited to, humans, non-human primates, farm animals, sport animals, rodents and pets. Non-limiting examples of non-human animal subjects include rodents such as mice, rats, hamsters, guinea pigs, rabbits, dogs, cats, sheep, pigs, goats, cattle, horses, apes and monkeys. In certain embodiments, the individual or subject is a human.

As used herein, a "label" refers to an agent that allows for direct or indirect detection. Labels include, but are not limited to, fluorescent labels, chromogenic labels, electron dense labels, chemiluminescent labels and radioactive labels. Non-limiting examples of labels include green fluorescent protein ("GFP"), mCherry, dtTomato, or other fluorescent proteins known in the art (e.g., Shaner et al., A Guide to Choosing Fluorescent Proteins, Nature Methods 2(12):905-909 (2005) , ³²P,¹⁴C,¹²⁵I, ³H and ¹³¹I, fluorogens (such as Rare Earth Chelate or lucifer yellow and its derivatives), Rhodamine (rhodamine) and its derivatives, dansyl, umbelliferone, luciferase (such as firefly luciferase and bacterial fluorescence plain enzyme) (U.S. Patent number 4,737,456), fluorescein, 2,3-dihydros phthalazine diketone, as well as enzymes producing detectable signals, *e.g.,* horseradish peroxidase (HRP), alkaline phosphatase enzyme, beta galactosidase, glucoamylase, lysozyme, carbohydrate oxidase (such as glucose oxidase, galactose oxidase and glucose-6-phosphate dehydrogenase (G6PD)) and heterocyclic oxidases (such as uricase and xanthine oxidase).

The term "ligation," as used herein, refers to the formation of a covalent bond or linkage between the termini of two or more nucleic acids.

The term "monoclonal antibody," as used herein, refers to an antibody obtained from a population of substantially homogeneous antibodies, *i.e.,* the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the presently disclosed subject matter may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The term "nucleic acid" or "polynucleotide" includes any compound and/or substance that comprises a polymer of nucleotides. Each nucleotide is composed of a base, specifically a purine- or pyrimidine base (*i.e.,* cytosine (C), guanine (G), adenine (A), thymine (T) or uracil (U)), a sugar (*i.e.,* deoxyribose or ribose), and a phosphate group. Often, the nucleic acid molecule is described by the sequence of bases, whereby said bases represent the primary structure (linear structure) of a nucleic acid molecule. The sequence of bases is typically represented from 5' to 3'. The term nucleic acid encompasses deoxyribonucleic acid (DNA) including, e.g., complementary DNA (cDNA) and genomic DNA, ribonucleic acid (RNA), *e.g.,* messenger RNA (mRNA), synthetic forms of DNA or RNA, and mixed polymers comprising two or more of these molecules. The nucleic acid molecule can be linear or circular. In addition, the term nucleic acid includes both sense and antisense strands, as well as single stranded and double stranded forms. Moreover, the herein described nucleic acid can contain naturally occurring or non-naturally occurring nucleotides. Examples of non-naturally occurring nucleotides include modified nucleotide bases with derivatized sugars or phosphate backbone linkages or chemically modified residues.

The term "plurality" refers to a number larger than one. In certain embodiments, the term "plurality of proteins" refers to a number of proteins larger than one. For example, but not by way of limitation, a plurality of proteins includes at least two proteins. In certain embodiments, the term "plurality of nucleic acids" refers to a number of nucleic acids larger than one. For example, but not by way of limitation, a plurality of nucleic acids includes at least two nucleic acids.

The term "reverse-transcription process" refers to a process of generating a complementary strand of DNA using an enzyme called a reverse transcriptase.

The term "sample," as used herein, refers to any sample containing one or more individual cells. In certain embodiments, "sample" refers to a sample of biological material obtained from a subject, *e.g.,* a tissue biopsy or a tissue sample). In certain embodiments, the sample can be obtained from a tissue, *e.g.,* a tissue sample. Non-limiting examples of tissues include eye, muscle, skin, tendon, vein, artery, blood, heart, spleen, lymph node, bone, bone marrow, lung, bronchi, trachea, gut, small intestine, large intestine, colon, rectum, salivary gland, tongue, gallbladder, appendix, liver, pancreas, brain, stomach, skin, kidney, ureter, bladder, urethra, gonad, testicle, ovary, uterus, fallopian tube, thymus, pituitary, thyroid, adrenal or parathyroid tissue. In certain embodiments, the samples are obtained from a subject. In certain embodiments, the subject can be a human, non-human primate, *e.g.,* an ape or a monkey, a farm animal, a mouse, a rat, a hamster, a guinea pig, a rabbit, a dog, cat, a sheep, a pig, a goat, a cow or a horse. In certain embodiments, the subject is a human. In certain embodiments, the sample can be obtained from preserved tissue, *e.g.,* fixed tissue, from frozen tissue or from fresh tissue, *e.g.,* tissue samples. In certain embodiments, a sample that can be analyzed using the methods of the present disclosure include at least two or more cells. For example, but not by way of limitation, a sample can include about 10 or more cells, about 100 or more cells, about 1,000 or more cells, about 5,000 or more cells, about 10,000 or more cells, about 20,000 or more cells, about 30,000 or more cells, about 40,000 or more cells, about 50,000 or more cells, about 100,000 or more cells, about 150,000 or more cells, about 200,000 or more cells, about 300,000 or more cells, about 400,000 or more cells or 500,000 or more cells.

The term "simultaneous," as used herein, is not limited to two actions occurring concurrently in time, but rather includes two actions occurring with respect to a single sample, e.g., staining a sample for the presence of one or more target protein(s) and subsequently staining the same sample for the presence of one or more target nucleic acid(s) results in the "simultaneous" staining of the sample for both the target nucleic(s) acid and the target protein(s).

The term "specifically binds," as used herein, refers to the preferential binding to a target molecule, *e.g.,* a protein or nucleic acid, relative to other molecules, *e.g.,* proteins or nucleic acids, in a sample.

### II. COMPOSITIONS AND METHODS FOR THE SIMULTANEOUS IMAGING OF NUCLEIC ACIDS AND PROTEINS

The present disclosure relates to compositions and methods for the simultaneous imaging of nucleic acids and proteins in a sample. The methods of the present disclosure can be put to a variety of uses, *e.g.,* the present disclosure provides methods for determining the spatial distribution of one or more proteins and one or more nucleic acids in a single sample. The methods of the present disclosure also allow for visualization of both a target nucleic acid *(e.g.,* a target DNA or a target RNA) and a target protein in the same sample. In certain embodiments, the sample used in the methods of the present disclosure can comprise a plurality of target nucleic acids, a plurality of target proteins, or a plurality of both target nucleic acids and target proteins. For example, but not by way of limitation, the methods of the present disclosure allow for the visualization of at least two or more target proteins (*e.g.,* at least three or more, at least four or more, at least five or more, at least six or more, at least seven or more, at least eight or more, at least nine or more or at least ten or more target proteins) and at least two or more target nucleic acids (*e.g.,* at least three or more, at least four or more, at least five or more, at least six or more, at least seven or more, at least eight or more, at least nine or more or at least ten or more target nucleic acids) in a single sample.

The compositions and methods of the present disclosure can also be used to determine particular characteristics of cells present in a sample. In certain embodiments, the one or more target proteins can be present in a different cell in the sample than the one or more target nucleic acids. In certain embodiments, each of the target proteins of the plurality of target proteins can be present in different cells within sample and/or each of the target nucleic acids of the plurality of target nucleic acids can be present in different cells within sample. For example, but not by way of limitation, compositions and methods of the present disclosure can be used to identify different cell types present in a sample.

The compositions and methods of the present disclosure allows for multimodal analyses over time to facilitate the detection of changes in cellular characteristics and/or the presence of different cell types in samples taken at independent time points. For example, but not by way of limitation, the present disclosure can include obtaining samples (*e.g.,* of a tissue) at different time points and performing a method of the present disclosure to visualize one or more target nucleic acids (*e.g.,* one or more target DNAs or RNAs) and one or more target proteins in the each of the samples to identify changes in the cellular characteristics of the tissue over time. In certain embodiments, the present disclosure can include obtaining samples (*e.g.,* of a tissue) at different time points and performing a method of the present disclosure to visualize one or more target nucleic acids (*e.g.*, one or more target DNAs or RNAs) and one or more target proteins in the each of the samples to identify changes in the type of cells present in the tissue over time.

The compositions and methods of the present disclosure can be used for diagnostic purposes. For example, but not by way of limitations, the compositions and methods of the present disclosure can be used to determine the presence and/or absence of protein variants and/or nucleic acid variants associated with a disease, *e.g.,* to diagnose a subject with a disease. In certain embodiments, the compositions and methods of the present disclosure can be used for pathological dissection of a tissue, *e.g.,* to identify a disease. In addition, the compositions and methods of the present disclosure allows for multimodal analyses over time to facilitate diagnostic assessments.

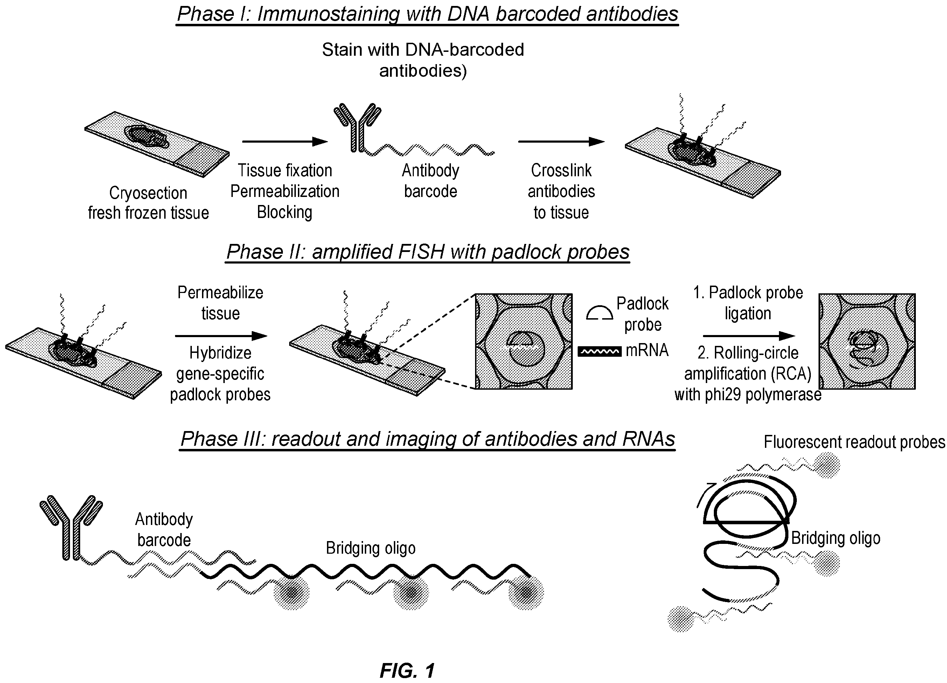

FIG. 1 provides a flowchart of an exemplary method of the present disclosure. In certain embodiments, a method of the present disclosure can include providing a sample, detecting one or more target proteins in the sample, detecting one or more target nucleic acids in the sample and imaging the one or more target proteins and the one or more target nucleic acids in the sample. In certain embodiments, the provision of a sample comprises the preparation of the sample.

### A. Sample Preparation

As shown in FIG. 1, the methods of the present disclosure include the preparation of a sample for the imaging of one or more target proteins and one or more target nucleic acids in the sample.

The sample to be analyzed can be prepared prior to detection of the one or more target proteins and the one or more target nucleic acids. Such sample preparation can comprise a fixation process, a permeabilization process, a dehydration process, a rehydration process, a post-fixation process and/or a nuclease inhibition process. In certain embodiments, sample preparation includes a fixation process. In certain embodiments, sample preparation comprises a fixation process and a dehydration process. In certain embodiments, sample preparation can further comprise a permeabilization process. Sample preparation can further include a rehydration process. Sample preparation can comprise a fixation process, a dehydration process and a rehydration process. Sample preparation can comprise a fixation process, a dehydration process, a permeabilization process and a rehydration process. Sample preparation can further include a post-fixation process. Sample preparation can comprise a fixation process, a dehydration process, a permeabilization process, a rehydration process and a post-fixation process. Sample preparation can further include a nuclease inhibition process. Sample preparation can comprise a fixation process, a dehydration process, a permeabilization process, a rehydration process, a post-fixation process and a nuclease inhibition process.

In certain embodiments, the fixation process comprises contacting a sample with a fixative. Non-limiting examples of fixatives include aldehydes (*e.g.,* formaldehyde, paraformaldehyde and glutaraldehyde), imidoesters, N-Hydroxysuccinimide (NHS) esters (*e.g.,* Bis-NHS ester), alcohols (*e.g.,* methanol and ethanol), acetone and acetic acid. The fixative can be formaldehyde. The fixative can include two or more fixatives. For example, the fixative can include formaldehyde and glutaraldehyde. The sample can be fixed in a final fixative concentration of about 0.1% to about 10%, about 1% to about 10%, about 1% to about 8%, about 2% to about 7%, about 3% to about 6% or about 3% to about 5%. The sample can be fixed in a final fixative concentration of about 3% to about 6%. The sample can be fixed in a final fixative concentration of about 3% to about 5%. The sample can be fixed in a final fixative concentration of about 4%. The sample can be fixed in a final formaldehyde concentration of about 0.1% to about 10%, about 1% to about 10%, about 1% to about 8%, about 2% to about 7%, about 3% to about 6% or about 3% to about 5%, *e.g.,* about 4%. The sample can be contacted with a fixative for about 5 hours or less, about 4 hours or less, about 3 hours or less, about 2 hours or less, about 60 minutes or less, about 50 minutes or less, about 40 minutes or less, about 30 minutes or less, about 20 minutes or less, about 10 minutes or less or about 5 minutes or less. The sample can be contacted with a fixative for about 5 minutes to about 1 hour, e.g., for about 5 minutes to about 30 minutes. The sample can be contacted with a fixative for about 5 minutes to about 1 hour. The sample can be contacted with a fixative for about 5 minutes to about 30 minutes. The sample can be contacted by a fixative at a temperature ranging from about 0°C to 50°C, *e.g.,* at room temperature (RT). The method can include a post-fixative process. For example, the sample can be fixed after the sample is contacted with the protein binding reagent but prior to detecting a target nucleic acid in the sample. Fixatives disclosed herein can be used to post-fix the sample. The sample can be post-fixed with a final fixative concentration of about 3% to about 6%, *e.g.,* post-fixed in a final fixative concentration of about 4%. Post-fix fixative can include two or more fixatives, e.g., formaldehyde (*e.g.,* at a concentration from about 3% to about 6%, *e.g.,* about 4%) and glutaraldehyde (*e.g.,* at a concentration from about 0.5% to about 2%, *e.g.,* about 1%).

Sample preparation can comprise a dehydration process. The dehydration process results in the reduction of the amount of water in the sample. Such dehydration can be achieved by contacting the sample with an alcohol, *e.g.,* an alcohol series. Dehydration can comprise contacting the sample with solutions of increasing alcohol content. For example, but not by way of limitation, the dehydration process can comprise contacting the sample with an ethanol series, where the sample is contacted with ethanol solutions of increasing concentration. The sample can be contacted with each concentration of alcohol (*e.g.,* ethanol) in the alcohol series *(e.g.,* ethanol series) for about 0.5 min to about 1 hour, *e.g.,* about 1 minute. The ethanol series can include 70%, 75%, 80%, 85%, 90%, 95% and/or 100% ethanol, *e.g.,* 70%, 85% and/or 100% ethanol. The ethanol series can include 70%, 85% and 100% ethanol. The dehydration process can include contacting the sample with 70% ethanol, subsequently contacting the sample with 85% ethanol and then contacting the sample with 100% ethanol. A sample can be contacted with an alcohol, *e.g.,* of an alcohol series, at a temperature ranging from about 0°C to 50°C, *e.g.,* at room temperature (RT). The sample can be contacted with each different concentration of alcohol, *e.g.,* ethanol, for about 0.5 minutes to about 1 hour, *e.g.,* for about 0.5 minutes to about 10 minutes, *e.g.,* about 1 minute.

The sample can be permeabilized prior to contacting the sample with a protein binding reagent used for detecting a target protein. For example, but not by way of limitation, the sample can be permeabilized after fixation of the sample and prior to contacting the sample with a protein binding reagent. Techniques for permeabilizing cells are known in the art and one of skill in the art would be able to assess the appropriateness of a particular technique for use in connection with the methods of the present disclosure. Non-limiting examples of reagents for permeabilizing cells include detergents (*e.g.,* saponin, Tween-20 and Triton X-100) and fixatives (*e.g.,* acetone, methanol and ethanol). For example, but not by way of limitation, the sample can be permeabilized with an alcohol, *e.g.,* methanol, and/or a detergent, *e.g.,* such as Triton X-100. The sample can be permeabilized with a detergent. The sample is permeabilized with a fixative. A reagent for permeabilization can be used at a concentration of about 0.1% to about 10%, *e.g.,* about 0.1% to about 10%, about 0.1% to about 10%, about 0.1% to about 10%, about 0.1% to about 10%, about 0.1% to about 10%, about 0.1% to about 10%, about 0.1% to about 10%. A reagent (*e.g.,* a detergent) for permeabilization can be used at a concentration of about 0.1% to about 1.0%, *e.g.,* 0.5%. Permeabilization can be performed by contacting the fixed sample with 0.5% Triton X-100. The sample can be contacted with a permeabilization reagent for about 5 hours or less, about 4 hours or less, about 3 hours or less, about 2 hours or less, about 60 minutes or less, about 50 minutes or less, about 40 minutes or less, about 30 minutes or less, about 20 minutes or less, about 10 minutes or less or about 5 minutes or less. The sample can be contacted with a permeabilization reagent, *e.g.,* Triton X-100, for about 20 minutes. A sample can be contacted with a permeabilization reagent, *e.g.,* Triton X-100, at a temperature ranging from about 0°C to 50°C, *e.g.,* at room temperature (RT).

### B. Protein Staining

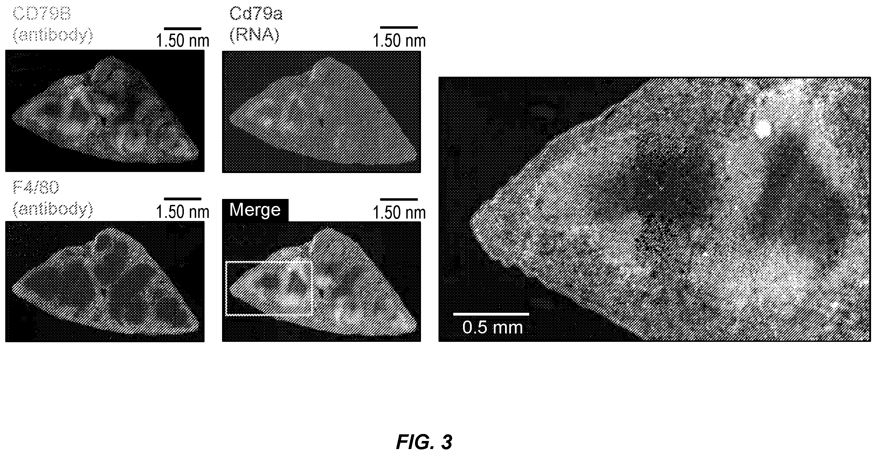

As shown in FIG. 1, the methods of the present disclosure can include the staining and imaging of one or more target proteins in a sample. For example, but not by way of limitation, methods of the present disclosure can include the staining and imaging of one target protein, two or more target proteins, three or more target proteins, four or more target proteins, five or more target proteins, six or more target proteins, seven or more target proteins, eight or more target proteins, nine or more target proteins or ten or more target proteins. At least 5 target proteins, at least 10 target proteins, at least 15 target proteins, at least 20 target proteins, at least 35 target proteins, at least 40 target proteins, at least 45 target proteins, at least 50 target proteins, at least 55 target proteins, at least 60 target proteins, at least 65 target proteins, at least 70 target proteins, at least 75 target proteins, at least 80 target proteins, at least 85 target proteins, at least 90 target proteins, at least 95 target proteins, at least 100 target proteins, at least 105 target proteins, at least 110 target proteins, at least 115 target proteins, at least 120 target proteins, at least 125 target proteins, at least 130 target proteins, at least 135 target proteins, at least 140 target proteins, at least 145 target proteins, at least 150 target proteins, at least 155 target proteins, at least 160 target proteins, at least 165 target proteins, at least 170 target proteins, at least 175 target proteins, at least 180 target proteins, at least 185 target proteins, at least 190 target proteins, at least 195 target proteins or at least 200 target proteins can be imaged in a single sample using the presently disclosed methods. About 15 to about 100 target proteins can be stained and imaged in a single sample using the presently disclosed methods. About 15 to about 30 target proteins can be stained and imaged in a single sample using the presently disclosed methods. The one or more target proteins can be present in the same cells within the sample. Alternatively, the one or more target proteins can be present within different cells (*e.g.,* different cell types) within the sample (*e.g.,* as shown in FIG. 3, FIG. 4 and FIG. 5).

Proteins that can be stained and imaged using the methods of the present disclosure include any protein that is present in or on the surface of a cell. For example, but not by way of limitation, the target protein can be an intracellular protein, an extracellular protein or a transmembrane protein. The target protein can be a mutated form of a protein or a wild type form of a protein. The target protein can be an exogenous protein, *e.g.,* a protein that is exogenously expressed in the sample. The target protein can be an endogenous protein, *e.g.,* a protein that is endogenously expressed in the sample. The target protein can be a post-translationally modified form of the protein.

The staining and imaging of one or more protein targets in a sample can comprise contacting a sample with a reagent that binds to a target protein, also referred to herein as a "protein binding reagent," in the sample. The protein binding reagent can be a reagent that specifically binds to a target protein, e.g., specifically binds to a target protein of a cell in a sample. The reagent that bind to the target protein allows for the imaging of the target protein. The reagent that binds to the target protein allows for the quantitative analysis of the target protein. Non-limiting example of protein binding reagents include antibodies (or antigen binding fragments thereof), aptamers, affimers, peptides and small molecules.

A protein binding reagent can be an antibody (or an antigen binding fragment thereof) that is specific for the target protein. The affinity between the antibody (or antigen binding fragment thereof) and the target protein is characterized by a dissociation constant (K_{d}) of ≤ 1 M, ≤ 100 mM, ≤ 10 mM, ≤ 1 mM, ≤ 100 µM, ≤ 10 µM, ≤ 1µM, ≤ 100 nM, ≤ 10 nM, ≤ 1 nM, ≤ 0.1 nM, ≤ 0.01 nM or ≤ 0.001 nM. The antibody specific for the target protein can have a K_{d} of about 10⁻³ or less, or 10⁻⁸ M or less, e.g., from 10⁻⁸ M to 10⁻¹³ M, *e.g.,* from 10⁻⁹ M to 10⁻¹³ M. The antibody can be an antibody fragment as described herein. For example, but not by way of limitation, the antibody can be a Fab, Fab', Fab'-SH, F(ab')₂, Fv, scFv, diabody or a single-domain antibody. The antibody can be a humanized or chimeric antibody.

The protein binding reagent can be coupled to an oligonucleotide. The antibody can be coupled to an oligonucleotide, which is also referred to herein as an "antibody-oligonucleotide conjugate." Exemplary antibody-oligonucleotide conjugates are shown and used in FIG. 1, FIG. 2, FIG. 4 and FIG 5. The oligonucleotide coupled to a protein binding reagent (e.g., an antibody or an antigen-binding fragment thereof) can be about 5 to about 200 nucleotides in length, *e.g.,* about 5 to about 150 nucleotides in length, about 5 to about 100 nucleotides in length, about 5 to about 50 nucleotides in length, about 10 to about 150 nucleotides in length, about 20 to about 100 nucleotides in length or about 10 to about 100 nucleotides in length. The oligonucleotide coupled to an antibody can be about 5 to about 50 nucleotides in length, *e.g.,* about 5 to about 45 nucleotides in length, about 5 to about 40 nucleotides in length, about 5 to about 35 nucleotides in length, about 5 to about 30 nucleotides in length, about 5 to about 25 nucleotides in length, about 5 to about 20 nucleotides in length, about 5 to about 15 nucleotides in length, about 5 to about 10 nucleotides in length, about 10 to about 50 nucleotides in length, about 15 to about 50 nucleotides in length, about 20 to about 50 nucleotides in length, about 25 to about 50 nucleotides in length, about 30 to about 50 nucleotides in length, about 35 to about 50 nucleotides in length, about 40 to about 50 nucleotides in length, about 10 to about 40 nucleotides in length or about 10 to about 30 nucleotides in length. The antibody can be a TotalSeq^{™} antibody (BioLegend, San Diego, CA). The use of an antibody-oligonucleotide conjugate allows for imaging of the protein by binding of a detection probe to the oligonucleotide conjugated to the antibody and/or to a bridging oligonucleotide that binds to the oligonucleotide conjugated to the antibody. The use of an antibody-oligonucleotide conjugate allows for imaging of the protein by amplification of the oligonucleotide conjugated to the antibody followed by detection of the resulting amplicon by binding of a detection probe to the amplicon and/or by detection of a bridging oligonucleotide that binds to the resulting amplicon. Alternatively, the use of an antibody-oligonucleotide conjugate allows for imaging of the protein by amplification of a blocking oligonucleotide (*e.g.,* an exonuclease blocking oligonucleotide) that binds to the oligonucleotide conjugated to the antibody followed by detection of the resulting amplicon by binding of a detection probe to the amplicon and/or by detection of a bridging oligonucleotide that binds to the resulting amplicon.

The oligonucleotide conjugated to the protein binding reagent can include a barcode, *e.g.,* as shown in FIG. 1. A barcode is a unique nucleotide sequence that can be used to identify the antibody coupled to the barcode. The barcode can be about 10 to about 50 nucleotides, *e.g.,* about 10 to about 30 nucleotides, in length. The barcode is about 10 to about 20 nucleotides in length. , the barcode is about 15 nucleotides in length. The detection probe can bind to the barcode, *e.g.,* the detection probe can comprise a sequence that is at least partially complementary to the barcode. Alternatively, or additionally, the detection probe can bind to a bridging oligonucleotide that binds the barcode, *e.g.,* the detection probe can comprise a sequence that is at least partially complementary to the bridging oligonucleotide that is at least partially complementary to the barcode, as shown in FIG. 1.

The oligonucleotide conjugated to the protein binding reagent can further include a primer sequence, *e.g.,* as shown in FIG. 1. The primer sequence can be used for amplification of the oligonucleotide. For example, but not by way of limitation, the primer sequence present in the oligonucleotide coupled to the protein binding reagent can be used to amplify the oligonucleotide during the amplification reaction used to amplify the target nucleic acid in the sample. The primer sequence can be about 10 to about 50 nucleotides, *e.g.,* about 10 to about 30 nucleotides, in length. The barcode can be about 20 nucleotides in length.

A plurality of protein binding reagents for binding multiple different target proteins can be used in the present disclosure. For example, but not by way of limitation, each protein binding reagent, *e.g.,* each antibody-oligonucleotide conjugate, specifically binds to a single target protein. Two or more protein binding reagents, three or more protein binding reagents, four or more protein binding reagents, five or more protein binding reagents, six or more protein binding reagents, seven or more protein binding reagents, eight or more protein binding reagents, nine or more protein binding reagents or ten or more protein binding reagents can be used in the present disclosure, where each protein binding reagent specifically binds to a single target protein. At least 5 protein binding reagents, at least 10 protein binding reagents, at least 15 protein binding reagents, at least 20 protein binding reagents, at least 35 protein binding reagents, at least 40 protein binding reagents, at least 45 protein binding reagents, at least 50 protein binding reagents, at least 55 protein binding reagents, at least 60 protein binding reagents, at least 65 protein binding reagents, at least 70 protein binding reagents, at least 75 protein binding reagents, at least 80 protein binding reagents, at least 85 protein binding reagents, at least 90 protein binding reagents, at least 95 protein binding reagents, at least 100 protein binding reagents, at least 105 protein binding reagents, at least 110 protein binding reagents, at least 115 protein binding reagents, at least 120 protein binding reagents, at least 125 protein binding reagents, at least 130 protein binding reagents, at least 135 protein binding reagents, at least 140 protein binding reagents, at least 145 protein binding reagents, at least 150 protein binding reagents, at least 155 protein binding reagents, at least 160 protein binding reagents, at least 165 protein binding reagents, at least 170 protein binding reagents, at least 175 protein binding reagents, at least 180 protein binding reagents, at least 185 protein binding reagents, at least 190 protein binding reagents, at least 195 protein binding reagents or at least 200 protein binding reagents can be used for detecting target proteins in a single sample by the methods of the present disclosure. About 15 to about 100 protein binding reagents can be used for detecting target proteins in a single sample by the methods of the present disclosure. About 15 to about 30 protein binding reagents can be used for detecting target proteins in a single sample by the methods of the present disclosure. The use of multiple protein binding reagents, *e.g.,* antibody-oligonucleotide conjugates, allows for the imaging of multiple proteins in a single sample, and can also allow for the analysis of the spatial location of each protein with respect to one another.

The sample can be contacted with the protein binding reagent, *e.g.,* the antibody specific for the target protein, for amount of time and under conditions to support specific binding of the protein binding reagent to the target protein. The sample can be contacted with the protein binding reagent, *e.g.,* the antibody specific for the target protein, for about 24 hours or less, about 23 hours or less, about 22 hours or less, about 21 hours or less, about 20 hours or less, about 19 hours or less, about 18 hours or less, about 17 hours or less, about 16 hours or less, about 15 hours or less, about 14 hours or less, about 13 hours or less, about 12 hours or less, about 11 hours or less, about 10 hours or less, about 9 hours or less, about 8 hours or less, about 7 hours or less, about 6 hours or less, about 5 hours or less, about 4 hours or less, about 3 hours or less, about 2 hours or less or about 60 minutes or less. The sample can be contacted with the protein binding reagent, *e.g.,* an antibody specific for the target protein, from about 1 to about 48 hours, e.g., from about 1 to about 42 hours, from about 1 to about 40 hours, from about 1 to about 38 hours, from about 1 to about 36 hours, from about 1 to about 34 hours, from about 1 to about 32 hours, from about 1 to about 30 hours, from about 1 to about 28 hours, from about 1 to about 26 hours, from about 1 to about 24 hours, from about 1 to about 22 hours, from about 1 to about 20 hours, from about 1 to about 18 hours, from about 1 to about 16 hours, from about 1 to about 14 hours, from about 1 to about 12 hours, from about 1 to about 10 hours, from about 1 to about 8 hours, from about 1 to about 6 hours, from about 1 to about 4 hours or from about 1 to about 2 hours. The sample can be contacted with the protein binding reagent, *e.g.,* an antibody specific for the target protein, for about 1 to about 24 hours. The sample can be contacted with the protein binding reagent, *e.g.,* an antibody specific for the target protein, for about 1 to about 20 hours. The sample can be contacted with the protein binding reagent, *e.g.,* an antibody specific for the target protein, for about 1 to about 16 hours. The sample can be contacted with the protein binding reagent, *e.g.,* an antibody specific for the target protein, for about 1 to about 12 hours. The sample can be contacted with the protein binding reagent, *e.g.,* an antibody specific for the target protein, for about 1 to about 8 hours. The sample can be contacted with the protein binding reagent, *e.g.,* antibody specific for the target protein, for about 1 to about 3 hours. A sample can be contacted with the protein binding reagent, *e.g.,* antibody specific for the target protein, at a temperature ranging from about 0°C to 50°C, *e.g.,* at room temperature (RT). A sample can be contacted with the protein binding reagent, *e.g.,* an antibody specific for the target protein, at a temperature ranging from about 0°C to 10°C, *e.g.,* at 4°C.

Contacting the protein binding reagent with the sample can include performing a blocking step to reduce background noise. The blocking step includes contacting the sample with one or more oligonucleotides, *e.g.,* blocking oligonucleotides, that are complementary to one or more nucleotide sequences, *e.g.,* two or more, three or more, four or more or five or more nucleotide sequences, present in the oligonucleotide conjugated to the antibody. The blocking oligonucleotide can be complementary to a conserved sequence present in the oligonucleotide coupled to the protein binding reagent, *e.g.,* a conserved sequence that is present in each oligonucleotide that is bound to the plurality of protein binding reagents used in the disclosed method. The blocking oligonucleotide is about 10 to about 100 nucleotides, *e.g.,* about 10 to about 50 nucleotides, in length. The blocking step can be performed prior to incubating the protein binding reagent with the sample. Alternatively, the blocking step can be performed at the same time as incubating the protein binding reagent with the sample. The blocking step can be performed after incubating the protein binding reagent with the sample. The blocking step can be performed for about 5 minutes to 24 hours, *e.g.,* for about 15 minutes to about 24 hours, about 15 minutes to about 20 hours, about 15 minutes to about 16 hours, about 15 minutes to about 12 hours, about 15 minutes to about 8 hours, about 15 minutes to about 4 hours, about 15 minutes to about 1 hour, from about 1 hour to about 24 hours, from about 2 hours to about 24 hours, from about 3 hours to about 24 hours, from about 4 hours to about 24 hours, from about 5 hours to about 24 hours, from about 6 hours to about 24 hours, from about 7 hours to about 24 hours, from about 8 hours to about 24 hours, from about 9 hours to about 24 hours, from about 10 hours to about 24 hours, from about 12 hours to about 24 hours, from about 14 hours to about 24 hours, from about 16 hours to about 24 hours, from about 18 hours to about 24 hours, from about 20 hours to about 24 hours, from about 22 hours to about 24 hours, from about 2 hours to about 12 hours or from about 2 hours to about 6 hours. The blocking step can be performed for about 1 to about 24 hours. The blocking step can be performed for about 1 to about 20 hours. The blocking step can be performed for about 1 to about 16 hours. The blocking step can be performed for about 1 to about 12 hours. The blocking step can be performed for about 1 to about 8 hours. The blocking step can be performed for about 1 to about 3 hours. The blocking step can be performed at a temperature ranging from about 0°C to 25°C, *e.g.,* at 4°C or at room temperature.

The samples to be analyzed by the methods of the present disclosure can undergo crosslinking. For example, but not by way of limitation, the samples can be crosslinked prior to detecting nucleic acids in the sample. Crosslinking of the sample can be achieved by exposing the cells to any crosslinking agent. Crosslinking can be performed using a fixative, as disclosed herein. Crosslinking can be performed by exposing the sample to formaldehyde. Crosslinking can be performed by exposing the sample to glutaraldehyde. Crosslinking can be performed by exposing the sample to a solution that includes formaldehyde and glutaraldehyde. Crosslinking can be performed by exposing the sample to a solution that includes from about 1% to about 10%, *e.g.,* about 4%, of paraformaldehyde and from about 0.1% to about 5%, *e.g.,* about 1%, of glutaraldehyde. Crosslinking is performed as a post-fixation step as described herein. Crosslinking is performed to maintain the binding of the target protein to the protein binding reagent during the sample treatment steps necessary for preparing the sample for nucleic acid detection. For example, but not by way of limitation, HCl used to permeabilize cells for nucleic acid detection abolishes binding of the target protein to the protein binding reagent and crosslinking prior to HCl treatment prevents this from occurring, thereby allowing simultaneous protein and nucleic acid detection in a single sample.

### C. Nucleic Acid Staining

Methods of the present disclosure can further include the staining and imaging of one or more nucleic acids in the sample. For example, but not by way of limitation, methods of the present disclosure can further include the staining and/or imaging of one or more nucleic acids in the sample before or after the staining and/or imaging of one or more proteins in the sample. Methods of the present disclosure can further include the staining of one or more nucleic acids in the sample before or after the staining of one or more proteins in the sample. Methods of the present disclosure can further include the imaging of one or more nucleic acids in the sample before or after the imaging of one or more proteins in the sample. Methods of the present disclosure can further include the staining of one or more nucleic acids in the sample after the staining of one or more proteins in the sample as described in Example 1. Methods of the present disclosure can further include the imaging of one or more nucleic acids in the sample after the imaging of one or more proteins in the sample as described in Example 1.

Methods of the present disclosure can further include the staining and imaging of one target nucleic acid, two or more target nucleic acids, three or more target nucleic acids, four or more target nucleic acids, five or more target nucleic acids, six or more target nucleic acids, seven or more target nucleic acids, eight or more target nucleic acids, nine or more target nucleic acids or ten or more target nucleic acids. At least 5 target nucleic acids, at least 10 target nucleic acids, at least 15 target nucleic acids, at least 20 target nucleic acids, at least 35 target nucleic acids, at least 40 target nucleic acids, at least 45 target nucleic acids, at least 50 target nucleic acids, at least 55 target nucleic acids, at least 60 target nucleic acids, at least 65 target nucleic acids, at least 70 target nucleic acids, at least 75 target nucleic acids, at least 80 target nucleic acids, at least 85 target nucleic acids, at least 90 target nucleic acids, at least 95 target nucleic acids or at least 100 target nucleic acids can be stained and imaged in a single sample using the presently disclosed methods. At least about 100 target nucleic acids, at least about 200 target nucleic acids, at least about 300 target nucleic acids, at least about 400 target nucleic acids, at least about 500 target nucleic acids, at least about 600 target nucleic acids, at least about 700 target nucleic acids, at least about 800 target nucleic acids, at least about 900 target nucleic acids, at least about 1,000 target nucleic acids, at least about 1,500 target nucleic acids, at least about 2,000 target nucleic acids, at least about 2,500 target nucleic acids, at least about 3,000 target nucleic acids, at least about 3,500 target nucleic acids, at least about 4,000 target nucleic acids, at least about 4,500 target nucleic acids or at least about 5,000 target nucleic acids can be stained and imaged in a single sample using the presently disclosed methods. About 100 target nucleic acids can be stained and imaged in a single sample using the presently disclosed methods.

A target nucleic acid can be any nucleic acid molecule (*e.g.,* a DNA molecule or an RNA molecule) present in the sample to be analyzed. The target nucleic acid can be an mRNA. The target nucleic acid can be a non-coding RNA, *e.g.,* a tRNA, an rRNA or a microRNA (miRNA). The target nucleic acid can be a DNA molecule. The target nucleic acid can be genomic DNA. The target nucleic acid can be an exogenous nucleic acid, *e.g.,* a viral nucleic acid. The target nucleic acid can be a variant of a target nucleic acid. The target nucleic acid can be an engineered barcode RNA. The target nucleic acid can be a guide RNA, *e.g.,* that can be used in a gene editing technique. The target nucleic acid can be a guide RNA for use with a CRISPR enzyme.

In certain embodiments, the target nucleic acid is the oligonucleotide coupled to the protein binding reagent and/or the blocking oligonucleotide, *e.g.,* the exonuclease blocking oligonucleotide, that is complementary to the oligonucleotide coupled to the protein binding reagent. For example, but not by way of limitation, the target nucleic acid is the oligonucleotide coupled to the protein binding reagent used in the method for detecting a target protein, *e.g.,* the target nucleic acid is the oligonucleotide of the antibody-oligonucleotide conjugate. The target nucleic acid can be the blocking oligonucleotide, *e.g.,* the exonuclease blocking oligonucleotide, that is complementary to the oligonucleotide coupled to the protein binding reagent. The target nucleic acid can be the blocking oligonucleotide, *e.g.,* the exonuclease blocking oligonucleotide, that is complementary to the oligonucleotide of the antibody-oligonucleotide conjugate.

The target nucleic acids to be stained and imaged using the disclosed methods can have varying lengths. A target nucleic acid can be about 10 or more, about 15 or more, about 20 or more, about 25 or more, about 30 or more, about 35 or more, about 40 or more, about 60 or more, about 80 or more, about 100 or more, about 150 or more, about 200 or more, about 300 or more, about 400 or more, about 500 or more, about 1,000 or more, about 5,000 or more or about 10,000 or more nucleotides in length. The target nucleic acid can include 10 or more consecutive nucleotides of a known sequence. For example, but not by way of limitation, a target nucleic acid can include about 10 or more, about 15 or more, about 20 or more, about 25 or more, about 30 or more, about 35 or more, about 40 or more, about 60 or more, about 80 or more, about 100 or more, about 150 or more, about 200 or more, about 300 or more, about 400 or more, about 500 or more, about 1,000 or more, about 5,000 or more or about 10,000 or more consecutive nucleotides of a known sequence. The target nucleic acid includes 10 or more consecutive nucleotides of an unknown sequence.

In certain embodiments, the staining and imaging of a nucleic acid in a sample comprises performance of an amplification process to amplify the target nucleic acid. Suitable nucleic acid amplification methods known in the art can be assessed by those of skill in the art to identify strategies appropriate to amplify the target nucleic acid. Non-limiting examples of such amplification methods include polymerase chain reaction (PCR), reverse transcriptase PCR, real-time PCR, rolling circle amplification (RCA), self-sustained sequence replication (3SR), nucleic acid sequence-based amplification (NASBA), strand displacement amplification (SDA), transcription-mediated amplification (TMA), single primer isothermal amplification (SPIA), helicase-dependent amplification (HDA), loop mediated amplification (LAMP), recombinase-polymerase amplification (RPA), nicking enzyme amplification reaction (NEAR), nicking endonuclease assisted nanoparticle activation (NENNA) and ligase chain reaction (LCR). Fakruddin et al., J. Pharm. Bioallied. Sci. 5(4): 245-252 (2013) and Yan et al., Mol. BioSyst. 10:970-1003 (2014) disclose additional amplification methods for use in the present disclosure, the contents of each of which are disclosed in their entireties herein. In certain embodiments, the amplification process is RCA.

The amplification process can include amplification reactions that use a polymerase with exonuclease activity, *e.g.,* a polymerase with 3'→5' exonuclease activity. The amplification reaction can include amplification reactions that include a polymerase with strand-displacement activity. Non-limiting examples of polymerases with strand-displacement and/or exonuclease activity include Bst polymerase, DNA polymerase ε (Pol ε), DNA polymerase δ (Pol δ) and a Phi29 polymerase and derivatives thereof. Derivatives of a Phi29 polymerase include Phi29 polymerases that comprise one or more modifications, *e.g.,* amino acid mutations, compared to a wild type Phi29 polymerase. The Phi29 polymerase is EquiPhi29^{™} (Thermo Scientific). The amplification process can include amplification reactions that include a Phi29 polymerase.

Performance of an amplification process includes contacting the sample with the reagents necessary for the amplification process and performing that process under conditions suitable for amplification of the target nucleic acid. Non-limiting examples of such reagents include polymerases, reverse transcriptases, nucleoside triphosphates or NTP analogues, primers, probes, primers, cofactors, ligation reaction reagents, endonucleases, lysis reagents, dyes, markers or labels. Additional reagents can include RNAse inhibitors to protect the integrity of the RNA in the sample, *e.g.,* by inhibiting the activity of RNase A, B and/or C.

In certain embodiments, nucleic acid staining and imaging in a sample can use padlock probes, *i.e.,* linear probes that can be converted into a circular DNA molecule by ligation upon hybridization to a target nucleic acid, *e.g.,* a target mRNA. In certain embodiments, ligation is performed using a ligase that ligates single stranded DNA, *e.g.,* SplintR ligase. Additional disclosure regarding the use of padlock oligonucleotides is provided in Sountoulidis et al., PLoS Biology 18(11):e3000675 (2020), the contents of which is herein incorporated by reference.

Hybridization of the padlock oligonucleotides to the target nucleic acids can occur in hybridization buffer for a period of about 1 hour to about 24 hours, *e.g.,* from about 2 hours to about 20 hours, from about 2 hours to about 16 hours or from about 2 hours to about 12 hours. Hybridization of the padlock oligonucleotides to the target nucleic acids can occur in a hybridization buffer for a period of about 2 hours to about 20 hours. Hybridization of the padlock oligonucleotides to the target nucleic acids can occur at a temperature ranging from about 20°C to about 60°C, *e.g.,* at about 40°C or about 45°C. Hybridization of the padlock oligonucleotides to the target nucleic acids can occur at a temperature ranging from about 30°C or about 50°C. The concentration of the oligonucleotides in the hybridization buffer can be from about 1 nM to about 1,000 nM, *e.g.,* from about 1 nM to about 900 nM, from about 1 nM to about 800 nM, from about 1 nM to about 700 nM, from about 1 nM to about 600 nM, from about 1 nM to about 500 nM, from about 1 nM to about 400 nM, from about 1 nM to about 300 nM, from about 1 nM to about 200 nM, from about 1 nM to about 100 nM or from about 1 nM to about 50 nM. The concentration of the oligonucleotides in the hybridization buffer can be from about 1 nM to about 50 nM. The hybridization buffer can include formamide, *e.g.,* about 10% to about 30% of formamide.

Ligation using a ligase, *e.g.,* ligation with the SplintR ligase, can be performed following the hybridization of the padlock oligonucleotides to the target nucleic acids. Ligation can occur for a period of about 1 hour to about 24 hours, e.g., from about 1 hours to about 20 hours, from about 1 hours to about 16 hours, from about 1 hour to about 12 hours, from about 5 hours to about 20 hours or from about 5 hours to about 16 hours. Ligation can occur for a period of about 1 hour to about 24 hours. Ligation can occur for a period of about 1 hour to about 20 hours. Ligation can occur for a period of about 1 hour to about 16 hours. Ligation can occur for a period of about 1 hour to about 12 hours. ligation can occur at a temperature ranging from about 10°C to 60°C, *e.g.,* at 25°C, 30°C or 40°C. ligation can occur at a temperature ranging from about 20°C to 50°C.

A plurality of oligonucleotides, e.g., padlock oligonucleotides, for detecting multiple different target nucleic acids can be used in the present disclosure. For example, but not by way of limitation, each oligonucleotide, *e.g.,* padlock oligonucleotide specifically binds to a single target nucleic acid. For example, but not by way of limitation, two or more padlock oligonucleotides, three or more padlock oligonucleotides, four or more padlock oligonucleotides, five or more padlock oligonucleotides, six or more padlock oligonucleotides, seven or more padlock oligonucleotides, eight or more padlock oligonucleotides, nine or more padlock oligonucleotides or ten or more padlock oligonucleotides can be used in the present disclosure, where each padlock oligonucleotide specifically binds to a single target nucleic acid. A method of the present disclosure can include contacting a sample with two or more, three or more, four or more, five or more, six or more, seven or more, eight or more, nine or more or ten or more padlock probes, where each padlock oligonucleotide specifically binds to a single target nucleic acid. One or more of the padlock oligonucleotides specifically binds to the oligonucleotide coupled to a protein binding reagent used in detecting a target protein in the sample. Two or more, three or more, four or more, five or more, six or more, seven or more, eight or more, nine or more or ten or more padlock probes can each bind to a distinct oligonucleotide of a protein binding reagent, *e.g.,* of a plurality of protein binding reagents.

Alternatively, and/or additionally, other techniques can be used in the present disclosure for detecting target nucleic acids. Other techniques that can be used in the present disclosure for detecting target nucleic acids include techniques that generate templates, *e.g.,* circular DNA templates, that can be amplified using a polymerase with strand-displacement activity, *e.g.,* a Phi29 polymerase or a derivative thereof. Other techniques that can be used in the present disclosure for detecting target nucleic acids include techniques that use amplification with a polymerase with strand-displacement activity, *e.g.,* Phi29 polymerase-based amplification. For example, but not by way of limitation, other techniques that can be used in the present disclosure for detecting target nucleic acids include SNAIL, RCP-FISH, DARTFISH and OPS. SNAIL includes the use of two oligonucleotides, where one oligonucleotide binds to the target nucleic acid and includes a ligation junction and the second oligonucleotide binds to the target nucleic acid and the ligation junction of the first oligonucleotide, followed by ligation to generate a circular DNA molecule. DARTFISH includes the generation of cDNA that is complementary to the target nucleic acid followed by the use of padlock probes that bind the cDNA and ligation to generate a circular DNA molecule.

The resulting circularized single-stranded DNA molecules can then be amplified using a polymerase with strand-displacement activity, *e.g.,* Phi29 polymerase or a derivative thereof, in an RCA process. This RCA process produces a single-stranded DNA molecule, referred to herein as an "RCA amplicon," containing multiple tandem repeats of the original target nucleic acid sequence. The RCA process can be performed for at least about 1 hour, at least about 5 hours, at least about 10 hours, at least about 15 hours, at least about 20 hours, at least about 21 hours, at least about 22 hours, at least about 23 hours or at least about 24 hours. The RCA process can be performed for at least about 24 hours. The RCA process can be performed for at least about 20 hours. The RCA process can be performed for at least about 16 hours. The RCA process can be performed for at least about 12 hours. The RCA process can be performed for at least about 24 hours. The RCA process can be performed for about 1 to about 24 hours, for about 1 to about 22 hours, for about 1 to about 20 hours, for about 1 to about 18 hours, for about 1 to about 16 hours, for about 1 to about 14 hours, for about 1 to about 12 hours, for about 1 to about 10 hours, for about 1 to about 8 hours, for about 1 to about 6 hours, for about 1 to about 4 hours or for about 1 to about 2 hours. The RCA process can be performed for about 8 to about 24 hours. The RCA process can be performed for about 12 to about 24 hours. The RCA process can be performed at a temperature ranging from about 0°C to 50°C, *e.g.,* at about 30°C.

The sample can be treated with a reagent that prevents degradation of the oligonucleotide conjugated to the protein binding reagent by preventing the exonuclease activity of the polymerase used in the amplification process. For example, but not by way of limitation, the sample can be treated with a reagent that prevents degradation of the oligonucleotide conjugated to the protein binding reagent by preventing the exonuclease activity, 3'→5' exonuclease activity, of a polymerase (*e.g.,* a polymerase with strand-displacement activity, *e.g.,* Phi29 polymerase or a derivative thereof ), that is used in the amplification process (*e.g.,* an RCA process). For example, but not by way of limitation, the sample can be treated with a reagent that prevents degradation of the oligonucleotide of the antibody-oligonucleotide conjugate (*e.g.,* the 3' end of the oligonucleotide of the antibody-oligonucleotide conjugate) by preventing the 3'→5' exonuclease activity of the Phi29 polymerase or a derivative thereof used in the amplification process (*e.g.,* the RCA process).

A blocking oligonucleotide, also referred to herein as a "Phi29 blocking oligonucleotide" or an "exonuclease blocking oligonucleotide," that binds to the oligonucleotide conjugated to the protein binding reagent can be used to prevent degradation of the oligonucleotide conjugated to the protein binding reagent by a polymerase with strand-displacement activity, *e.g.,* a Phi29 polymerase or a derivative thereof. The sample can be contacted with the exonuclease blocking oligonucleotide prior to performing the amplification process. The sample can be contacted with the exonuclease blocking oligonucleotide prior to contacting the sample with the polymerase with strand-displacement activity. The sample can be contacted with the exonuclease blocking oligonucleotide after binding of the protein binding reagent but before contacting the sample with the polymerase having strand-displacement activity and/or 3'→5' exonuclease activity. The sample can be contacted with the exonuclease blocking oligonucleotide prior to contacting the sample with a Phi29 polymerase.

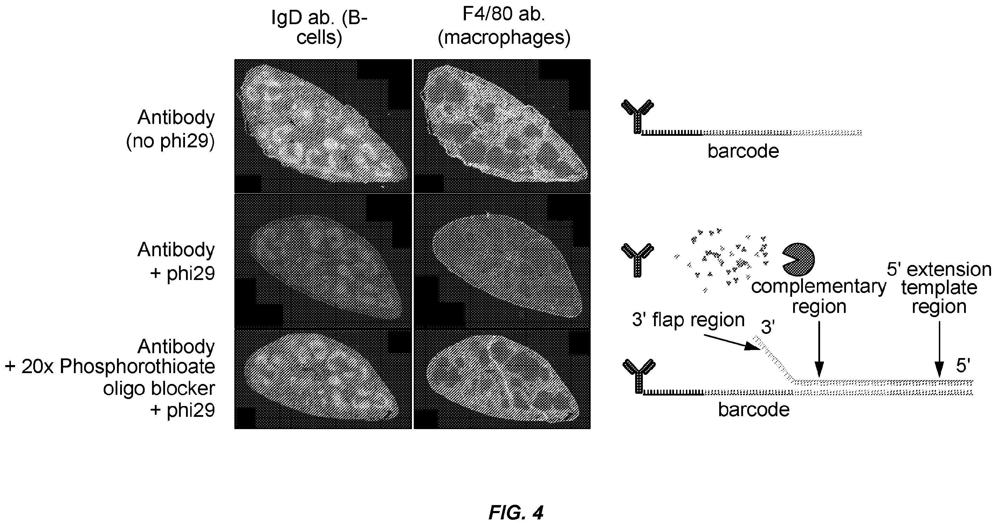

The exonuclease blocking oligonucleotide has the structure shown in FIG. 4. The exonuclease blocking oligonucleotide includes a sequence that is complementary to the oligonucleotide conjugated to the protein binding reagent. For example, but not by way of limitation, the exonuclease blocking oligonucleotide can include a sequence that is complementary to a region adjacent to the barcode sequence present in the oligonucleotide conjugated to the protein binding reagent as shown in FIG. 4. This region is located 3' from the barcode sequence of the oligonucleotide conjugated to the protein binding reagent.

The exonuclease blocking oligonucleotide does not bind to the barcode sequence of the oligonucleotide, *e.g.,* as shown in FIG. 4. The exonuclease blocking oligonucleotide does not include a nucleotide sequence that is complementary to the barcode sequence of the oligonucleotide. The exonuclease blocking oligonucleotide does not bind to the primer sequence of the oligonucleotide, *e.g.,* as shown in FIG. 4. The exonuclease blocking oligonucleotide does not include a nucleotide sequence that is complementary to the primer sequence of the oligonucleotide. The exonuclease blocking oligonucleotide includes a nucleotide sequence at its 3' end that does not bind to (*e.g.,* is not complementary to) a sequence of the oligonucleotide, *e.g.,* as shown in FIG. 4.

The exonuclease blocking oligonucleotide has a structure from 5' to 3' that includes (i) a nucleotide sequence (*e.g.,* a first nucleotide sequence *e.g.,* a complementary region or domain) that is complementary to the oligonucleotide conjugated to the protein binding reagent and (ii) a nucleotide sequence (*e.g.,* a second nucleotide sequence, *e.g.,* a 3' flap region or domain) that is not complementary to the oligonucleotide conjugated to the protein binding reagent. The exonuclease blocking oligonucleotide has a structure from 5' to 3' that includes (i) a nucleotide sequence (*e.g.,* a first nucleotide sequence, *e.g.,* a 5' extension template region or domain) comprising an extension template, (ii) a nucleotide sequence (*e.g.,* a second nucleotide sequence, *e.g.,* a complementary region or domain) that is complementary to the oligonucleotide conjugated to the protein binding reagent and (iii) a nucleotide sequence (*e.g.,* a third nucleotide sequence, *e.g.,* a 3' flap region or domain) that is not complementary to the oligonucleotide conjugated to the protein binding reagent, *e.g.,* as shown in FIG. 4. The nucleotide sequence (*e.g.*, the first nucleotide sequence) comprising the extension template is not complementary to the oligonucleotide conjugated to the protein binding reagent. The nucleotide sequence of the oligonucleotide coupled to the protein binding reagents that is bound by the exonuclease blocking oligonucleotide can be conserved among each oligonucleotide coupled to the plurality of protein binding reagents used in a method of the present disclosure.

The exonuclease blocking oligonucleotide can further include an extension template at its 5' end. The extension template at the 5' end of the exonuclease blocking oligonucleotide can prevent exonuclease activity of a polymerase, *e.g.,* a polymerase with strand-displacement activity, *e.g.,* a Phi29 polymerase. The extension template at the 5' end of the exonuclease blocking oligonucleotide can prevent 3'→5' exonuclease activity of the polymerase on single-stranded nucleic acids by generating a double-stranded nucleic acid. The extension template at the 5' end of the exonuclease blocking oligonucleotide can prevent exonuclease activity of a polymerase, *e.g.,* a polymerase with strand-displacement activity, *e.g.,* a Phi29 polymerase, on the oligonucleotide of protein binding reagent-oligonucleotide conjugate. The extension template at the 5' end of the exonuclease blocking oligonucleotide can prevent exonuclease activity of the Phi29 polymerase on the oligonucleotide of the protein binding reagent-oligonucleotide conjugate.

The exonuclease blocking oligonucleotide can be about 5 to about 200 nucleotides in length, e.g., about 5 to about 150 nucleotides in length, about 5 to about 100 nucleotides in length, about 5 to about 50 nucleotides in length, about 10 to about 150 nucleotides in length, about 20 to about 100 nucleotides in length or about 10 to about 100 nucleotides in length. The exonuclease blocking oligonucleotide is about 10 to about 100 nucleotides in length. The exonuclease blocking oligonucleotide can be about 20 to about 90 nucleotides in length. The exonuclease blocking oligonucleotide can be about 30 to about 90 nucleotides in length. The exonuclease blocking oligonucleotide can be about 40 to about 90 nucleotides in length. The exonuclease blocking oligonucleotide can be about 50 to about 90 nucleotides in length. The exonuclease blocking oligonucleotide can be about 60 to about 90 nucleotides in length. The exonuclease blocking oligonucleotide is about 20 to about 80 nucleotides in length. The exonuclease blocking oligonucleotide can be about 30 to about 80 nucleotides in length. The exonuclease blocking oligonucleotide can be about 40 to about 80 nucleotides in length. The exonuclease blocking oligonucleotide can be about 50 to about 80 nucleotides in length. The exonuclease blocking oligonucleotide can be about 60 to about 80 nucleotides in length. The exonuclease blocking oligonucleotide can be about 45 to about 75 nucleotides in length. The exonuclease blocking oligonucleotide can be about 30 to about 60 nucleotides in length. The exonuclease blocking oligonucleotide can be about 5 to about 50 nucleotides in length, *e.g.,* about 5 to about 45 nucleotides in length, about 5 to about 40 nucleotides in length, about 5 to about 35 nucleotides in length, about 5 to about 30 nucleotides in length, about 5 to about 25 nucleotides in length, about 5 to about 20 nucleotides in length, about 5 to about 15 nucleotides in length, about 5 to about 10 nucleotides in length, about 10 to about 50 nucleotides in length, about 15 to about 50 nucleotides in length, about 20 to about 50 nucleotides in length, about 25 to about 50 nucleotides in length, about 30 to about 50 nucleotides in length, about 35 to about 50 nucleotides in length, about 40 to about 50 nucleotides in length, about 10 to about 40 nucleotides in length or about 10 to about 30 nucleotides in length. The exonuclease blocking oligonucleotide can be about 30 to about 50 nucleotides in length.

The nucleotide sequence of the exonuclease blocking oligonucleotide that binds to the oligonucleotide of the protein binding reagent (*e.g.,* that is complementary to the oligonucleotide of the protein binding reagent) can be about 5 to about 100 nucleotides in length (e.g., where the nucleotides are consecutive). For example, but not by way of limitation, the nucleotide sequence of the exonuclease blocking oligonucleotide that binds to the oligonucleotide of the protein binding reagent (*e.g.,* that is complementary to the oligonucleotide of the protein binding reagent) is about 5 to about 95 nucleotides in length, about 5 to about 90 nucleotides in length, about 5 to about 85 nucleotides in length, about 5 to about 80 nucleotides in length, about 5 to about 75 nucleotides in length, about 5 to about 70 nucleotides in length, about 5 to about 65 nucleotides in length, about 5 to about 60 nucleotides in length, about 5 to about 55 nucleotides in length, about 5 to about 50 nucleotides in length, about 5 to about 45 nucleotides in length, about 5 to about 40 nucleotides in length, about 5 to about 35 nucleotides in length, about 5 to about 30 nucleotides in length, about 5 to about 25 nucleotides in length, about 5 to about 20 nucleotides in length, about 5 to about 15 nucleotides in length, about 5 to about 10 nucleotides in length, about 10 to about 100 nucleotides in length, about 15 to about 100 nucleotides in length, about 20 to about 100 nucleotides in length, about 25 to about 100 nucleotides in length, about 30 to about 100 nucleotides in length, about 35 to about 100 nucleotides in length, about 40 to about 100 nucleotides in length, about 45 to about 100 nucleotides in length, about 50 to about 100 nucleotides in length, about 55 to about 100 nucleotides in length, about 60 to about 100 nucleotides in length, about 65 to about 100 nucleotides in length, about 70 to about 100 nucleotides in length, about 75 to about 100 nucleotides in length, about 80 to about 100 nucleotides in length, about 85 to about 100 nucleotides in length, about 90 to about 100 nucleotides in length, about 95 to about 100 nucleotides in length, about 30 to about 90 nucleotides in length, about 30 to about 80 nucleotides in length, about 30 to about 70 nucleotides in length, about 30 to about 60 nucleotides in length, about 30 to about 50 nucleotides in length, about 10 to about 40 nucleotides in length or about 10 to about 30 nucleotides in length. The nucleotide sequence of the exonuclease blocking oligonucleotide that binds to the oligonucleotide of the protein binding reagent (*e.g.,* that is complementary to the oligonucleotide of the protein binding reagent) can be about 5 to about 50 nucleotides in length. The nucleotide sequence of the exonuclease blocking oligonucleotide that binds to the oligonucleotide of the protein binding reagent (*e.g.,* that is complementary to the oligonucleotide of the protein binding reagent) can be about 5 to about 40 nucleotides in length. The nucleotide sequence of the exonuclease blocking oligonucleotide that binds to the oligonucleotide of the protein binding reagent (*e.g.,* that is complementary to the oligonucleotide of the protein binding reagent) can be about 5 to about 30 nucleotides in length. The nucleotide sequence of the exonuclease blocking oligonucleotide that binds to the oligonucleotide of the protein binding reagent (*e.g.,* that is complementary to the oligonucleotide of the protein binding reagent) can be about 10 to about 50 nucleotides in length. The nucleotide sequence of the exonuclease blocking oligonucleotide that binds to the oligonucleotide of the protein binding reagent (*e.g.,* that is complementary to the oligonucleotide of the protein binding reagent) can be about 10 to about 40 nucleotides in length. The nucleotide sequence of the exonuclease blocking oligonucleotide that binds to the oligonucleotide of the protein binding reagent (*e.g.,* that is complementary to the oligonucleotide of the protein binding reagent) can be about 20 to about 30 nucleotides in length. The nucleotide sequence of the exonuclease blocking oligonucleotide that binds to the oligonucleotide of the protein binding reagent can be about 50 to about 100 nucleotides in length. The nucleotide sequence of the exonuclease blocking oligonucleotide that binds to the oligonucleotide of the protein binding reagent can be about 60 to about 100 nucleotides in length. The nucleotide sequence of the exonuclease blocking oligonucleotide that binds to the oligonucleotide of the protein binding reagent can be about 70 to about 100 nucleotides in length. The nucleotide sequence of the exonuclease blocking oligonucleotide that binds to the oligonucleotide of the protein binding reagent can be about 80 to about 100 nucleotides in length. The nucleotide sequence of the exonuclease blocking oligonucleotide that binds to the oligonucleotide of the protein binding reagent can be about 90 to about 100 nucleotides in length.

The nucleotide sequence of the exonuclease blocking oligonucleotide that binds to the oligonucleotide of the protein binding reagent can be at least about 90%, at least about 91%, at least about 92%, at least about 93%, at least about 94%, at least about 95%, at least about 96%, at least about 97%, at least about 98%, at least about 99% or about 100% complementary to a nucleotide sequence of the oligonucleotide of the protein binding reagent. The nucleotide sequence of the exonuclease blocking oligonucleotide that binds to the oligonucleotide of the protein binding reagent can be at least about 90% complementary to a nucleotide sequence of the oligonucleotide of the protein binding reagent. The nucleotide sequence of the exonuclease blocking oligonucleotide that binds to the oligonucleotide of the protein binding reagent can be at least about 95% complementary to a nucleotide sequence of the oligonucleotide of the protein binding reagent. The nucleotide sequence of the exonuclease blocking oligonucleotide that binds to the oligonucleotide of the protein binding reagent can be at least about 97% complementary to a nucleotide sequence of the oligonucleotide of the protein binding reagent. The nucleotide sequence of the exonuclease blocking oligonucleotide that binds to the oligonucleotide of the protein binding reagent can be at least about 98% complementary to a nucleotide sequence of the oligonucleotide of the protein binding reagent. The nucleotide sequence of the exonuclease blocking oligonucleotide that binds to the oligonucleotide of the protein binding reagent can be at least about 99% complementary to a nucleotide sequence of the oligonucleotide of the protein binding reagent. , the nucleotide sequence of the exonuclease blocking oligonucleotide that binds to the oligonucleotide of the protein binding reagent is 100% complementary to a nucleotide sequence of the oligonucleotide of the protein binding reagent.

About 10% to about 90% of the nucleotide sequence of the exonuclease blocking oligonucleotide can be complementary to the oligonucleotide of the protein binding reagent. For example, but not by way of limitation, about 15% to about 90%, about 20% to about 90%, about 25% to about 90%, about 30% to about 90%, about 35% to about 90%, about 40% to about 45%, about 50% to about 90%, about 55% to about 90%, about 60% to about 90%, about 65% to about 90%, about 70% to about 90%, about 80% to about 90%, about 85% to about 90%, about 10% to about 85%, about 10% to about 80%, about 10% to about 75%, about 10% to about 70%, about 10% to about 65%, about 10% to about 60%, about 10% to about 55%, about 10% to about 50%, about 10% to about 45%, about 10% to about 40%, about 10% to about 35%, about 10% to about 30%, about 10% to about 25%, about 10% to about 20%, about 20% to about 80%, about 20% to about 70%, about 20% to about 60%, about 30% to about 80%, about 30% to about 70%, about 30% to about 60%, about 40% to about 80%, about 40% to about 70%, about 40% to about 60%, about 50% to about 80%, about 50% to about 70%, about 50% to about 60%, about 60% to about 80% or about 60% to about 70% of the nucleotide sequence of the exonuclease blocking oligonucleotide is complementary to the oligonucleotide of the protein binding reagent. About 50% to about 90% of the nucleotide sequence of the exonuclease blocking oligonucleotide can be complementary to the oligonucleotide of the protein binding reagent. About 60% to about 90% of the nucleotide sequence of the exonuclease blocking oligonucleotide can be complementary to the oligonucleotide of the protein binding reagent. About 70% to about 90% of the nucleotide sequence of the exonuclease blocking oligonucleotide can be complementary to the oligonucleotide of the protein binding reagent. About 80% to about 90% of the nucleotide sequence of the exonuclease blocking oligonucleotide can be complementary to the oligonucleotide of the protein binding reagent. About 20% to about 60% of the nucleotide sequence of the exonuclease blocking oligonucleotide can be complementary to the oligonucleotide of the protein binding reagent. About 10% to about 50% of the nucleotide sequence of the exonuclease blocking oligonucleotide can be complementary to the oligonucleotide of the protein binding reagent. About 20% to about 60% of the nucleotide sequence of the exonuclease blocking oligonucleotide can be complementary to the oligonucleotide of the protein binding reagent. About 30% to about 60% of the nucleotide sequence of the exonuclease blocking oligonucleotide can be complementary to the oligonucleotide of the protein binding reagent. About 40% to about 60% of the nucleotide sequence of the exonuclease blocking oligonucleotide v complementary to the oligonucleotide of the protein binding reagent. , about 50% to about 60% of the nucleotide sequence of the exonuclease blocking oligonucleotide is complementary to the oligonucleotide of the protein binding reagent. About 20% to about 50% of the nucleotide sequence of the exonuclease blocking oligonucleotide can be complementary to the oligonucleotide of the protein binding reagent. About 30% to about 50% of the nucleotide sequence of the exonuclease blocking oligonucleotide can be complementary to the oligonucleotide of the protein binding reagent. About 40% to about 50% of the nucleotide sequence of the exonuclease blocking oligonucleotide can be complementary to the oligonucleotide of the protein binding reagent. About 30% to about 40% of the nucleotide sequence of the exonuclease blocking oligonucleotide can be complementary to the oligonucleotide of the protein binding reagent.

The nucleotide sequence of the exonuclease blocking oligonucleotide that comprises the extension template can be about 5 to about 100 nucleotides in length (*e.g.,* where the nucleotides are consecutive). For example, but not by way of limitation, the nucleotide sequence of the exonuclease blocking oligonucleotide that comprises the extension template can be about 5 to about 95 nucleotides in length, about 5 to about 90 nucleotides in length, about 5 to about 85 nucleotides in length, about 5 to about 80 nucleotides in length, about 5 to about 75 nucleotides in length, about 5 to about 70 nucleotides in length, about 5 to about 65 nucleotides in length, about 5 to about 60 nucleotides in length, about 5 to about 55 nucleotides in length, about 5 to about 50 nucleotides in length, about 5 to about 45 nucleotides in length, about 5 to about 40 nucleotides in length, about 5 to about 35 nucleotides in length, about 5 to about 30 nucleotides in length, about 5 to about 25 nucleotides in length, about 5 to about 20 nucleotides in length, about 5 to about 15 nucleotides in length, about 5 to about 10 nucleotides in length, about 10 to about 100 nucleotides in length, about 15 to about 100 nucleotides in length, about 20 to about 100 nucleotides in length, about 25 to about 100 nucleotides in length, about 30 to about 100 nucleotides in length, about 35 to about 100 nucleotides in length, about 40 to about 100 nucleotides in length, about 45 to about 100 nucleotides in length, about 50 to about 100 nucleotides in length, about 55 to about 100 nucleotides in length, about 60 to about 100 nucleotides in length, about 65 to about 100 nucleotides in length, about 70 to about 100 nucleotides in length, about 75 to about 100 nucleotides in length, about 80 to about 100 nucleotides in length, about 85 to about 100 nucleotides in length, about 90 to about 100 nucleotides in length, about 95 to about 100 nucleotides in length, about 30 to about 90 nucleotides in length, about 30 to about 80 nucleotides in length, about 30 to about 70 nucleotides in length, about 30 to about 60 nucleotides in length, about 30 to about 50 nucleotides in length, about 10 to about 40 nucleotides in length or about 10 to about 30 nucleotides in length. The nucleotide sequence of the exonuclease blocking oligonucleotide that comprises the extension template can be about 50 to about 100 nucleotides in length. The nucleotide sequence of the exonuclease blocking oligonucleotide that comprises the extension template can be about 60 to about 100 nucleotides in length. The nucleotide sequence of the exonuclease blocking oligonucleotide that comprises the extension template can be about 70 to about 100 nucleotides in length. The nucleotide sequence of the exonuclease blocking oligonucleotide that comprises the extension template can be about 80 to about 100 nucleotides in length. The nucleotide sequence of the exonuclease blocking oligonucleotide that comprises the extension template can be about 90 to about 100 nucleotides in length. The nucleotide sequence of the exonuclease blocking oligonucleotide that comprises the extension template can be about 20 to about 60 nucleotides in length. The nucleotide sequence of the exonuclease blocking oligonucleotide that comprises the extension template can be about 20 to about 50 nucleotides in length. The nucleotide sequence of the exonuclease blocking oligonucleotide that comprises the extension template can be about 20 to about 40 nucleotides in length. The nucleotide sequence of the exonuclease blocking oligonucleotide that comprises the extension template can be about 20 to about 30 nucleotides in length. The nucleotide sequence of the exonuclease blocking oligonucleotide that comprises the extension template can be about 10 to about 40 nucleotides in length. The nucleotide sequence of the exonuclease blocking oligonucleotide that comprises the extension template can be about 10 to about 30 nucleotides in length. The nucleotide sequence of the exonuclease blocking oligonucleotide that comprises the extension template can be about 10 to about 20 nucleotides in length. The nucleotide sequence of the exonuclease blocking oligonucleotide that comprises the extension template can be about 20 to about 30 nucleotides in length.

The nucleotide sequence of the exonuclease blocking oligonucleotide that does not bind to the oligonucleotide of the protein binding reagent (*e.g.,* that is not complementary to the oligonucleotide of the protein binding reagent) can be about 5 to about 100 nucleotides in length (*e.g.,* where the nucleotides are consecutive). The nucleotide sequence at the 3' end of the exonuclease blocking oligonucleotide that does not bind to the oligonucleotide of the protein binding reagent (*e.g.,* that is not complementary to the oligonucleotide of the protein binding reagent) can be about 5 to about 100 nucleotides in length (*e.g.,* where the nucleotides are consecutive). For example, but not by way of limitation, the nucleotide sequence of the exonuclease blocking oligonucleotide that does not bind to the oligonucleotide of the protein binding reagent (*e.g.,* that is not complementary to the oligonucleotide of the protein binding reagent) can be about 5 to about 95 nucleotides in length, about 5 to about 90 nucleotides in length, about 5 to about 85 nucleotides in length, about 5 to about 80 nucleotides in length, about 5 to about 75 nucleotides in length, about 5 to about 70 nucleotides in length, about 5 to about 65 nucleotides in length, about 5 to about 60 nucleotides in length, about 5 to about 55 nucleotides in length, about 5 to about 50 nucleotides in length, about 5 to about 45 nucleotides in length, about 5 to about 40 nucleotides in length, about 5 to about 35 nucleotides in length, about 5 to about 30 nucleotides in length, about 5 to about 25 nucleotides in length, about 5 to about 20 nucleotides in length, about 5 to about 15 nucleotides in length, about 5 to about 10 nucleotides in length, about 10 to about 100 nucleotides in length, about 15 to about 100 nucleotides in length, about 20 to about 100 nucleotides in length, about 25 to about 100 nucleotides in length, about 30 to about 100 nucleotides in length, about 35 to about 100 nucleotides in length, about 40 to about 100 nucleotides in length, about 45 to about 100 nucleotides in length, about 50 to about 100 nucleotides in length, about 55 to about 100 nucleotides in length, about 60 to about 100 nucleotides in length, about 65 to about 100 nucleotides in length, about 70 to about 100 nucleotides in length, about 75 to about 100 nucleotides in length, about 80 to about 100 nucleotides in length, about 85 to about 100 nucleotides in length, about 90 to about 100 nucleotides in length, about 95 to about 100 nucleotides in length, about 30 to about 90 nucleotides in length, about 30 to about 80 nucleotides in length, about 30 to about 70 nucleotides in length, about 30 to about 60 nucleotides in length, about 30 to about 50 nucleotides in length, about 10 to about 40 nucleotides in length or about 10 to about 30 nucleotides in length. The nucleotide sequence of the exonuclease blocking oligonucleotide that does not bind to the oligonucleotide of the protein binding reagent can be about 50 to about 100 nucleotides in length. The nucleotide sequence of the exonuclease blocking oligonucleotide that does not bind to the oligonucleotide of the protein binding reagent can be about 60 to about 100 nucleotides in length. The nucleotide sequence of the exonuclease blocking oligonucleotide that does not bind to the oligonucleotide of the protein binding reagent can be about 70 to about 100 nucleotides in length. The nucleotide sequence of the exonuclease blocking oligonucleotide that does not bind to the oligonucleotide of the protein binding reagent can be about 80 to about 100 nucleotides in length. The nucleotide sequence of the exonuclease blocking oligonucleotide that does not bind to the oligonucleotide of the protein binding reagent can be about 90 to about 100 nucleotides in length. The nucleotide sequence of the exonuclease blocking oligonucleotide that does not bind to the oligonucleotide of the protein binding reagent can be about 20 to about 80 nucleotides in length. The nucleotide sequence of the exonuclease blocking oligonucleotide that does not bind to the oligonucleotide of the protein binding reagent can be about 20 to about 60 nucleotides in length. The nucleotide sequence of the exonuclease blocking oligonucleotide that does not bind to the oligonucleotide of the protein binding reagent can be about 20 to about 50 nucleotides in length. The nucleotide sequence of the exonuclease blocking oligonucleotide that does not bind to the oligonucleotide of the protein binding reagent can be about 25 to about 45 nucleotides in length. The nucleotide sequence of the exonuclease blocking oligonucleotide that does not bind to the oligonucleotide of the protein binding reagent can be about 5 to about 40 nucleotides in length. The nucleotide sequence of the exonuclease blocking oligonucleotide (e.g., at the 3' end) that does not bind to the oligonucleotide of the protein binding reagent can be about 5 to about 35 nucleotides in length. The nucleotide sequence of the exonuclease blocking oligonucleotide (e.g., at the 3' end) that does not bind to the oligonucleotide of the protein binding reagent can be about 5 to about 30 nucleotides in length. The nucleotide sequence of the exonuclease blocking oligonucleotide (e.g., at the 3' end) that does not bind to the oligonucleotide of the protein binding reagent is about 5 to about 25 nucleotides in length. the nucleotide sequence of the exonuclease blocking oligonucleotide (e.g., at the 3' end) that does not bind to the oligonucleotide of the protein binding reagent is about 5 to about 20 nucleotides in length. the nucleotide sequence of the exonuclease blocking oligonucleotide (e.g., at the 3' end) that does not bind to the oligonucleotide of the protein binding reagent is about 10 to about 20 nucleotides in length.

The exonuclease blocking oligonucleotide can include one or more modified nucleotides. The exonuclease blocking oligonucleotide can include at least 5 or more, at least 6 or more, at least 7 or more, at least 8 or more, at least 9 or more, at least 10 or more, at least 11 or more, at least 12 or more, at least 13 or more, at least 14 or more, at least 15 or more, at least 16 or more, at least 17 or more, at least 18 or more, at least 19 or more or at least 20 or more modified nucleotides. The exonuclease blocking oligonucleotide can include from about 5 to about 20 modified nucleotides. The exonuclease blocking oligonucleotide can include from about 5 to about 15 modified nucleotides. The exonuclease blocking oligonucleotide can include from about 10 to about 15 modified nucleotides. The modified nucleotide can be a nucleotide that is resistant to exonuclease cleavage. The modified nucleotide can include modified nucleotide bases with derivatized sugars or phosphate backbone linkages or chemically modified residues. the modified nucleotide is a nucleotide with a phosphate backbone modification. The modified nucleotide can be a nucleotide with a phosphorothioate linkage at its 3' end. The modified nucleotide can be a 2',3'-dideoxynucleoside-alpha-thiol nucleotide, e.g., a 2',3'-dideoxyadenosine-5'-O-(1-thiotriphosphate), 2',3'-dideoxycytidine-5'-O-(1-thiotriphosphate), 2',3'-dideoxyguanosine-5'-O-(1-thiotriphosphate) and/or 2',3'-dideoxythymidine-5'-O-(1-thiotriphosphate). The exonuclease blocking oligonucleotide can include at least 5 or more, at least 6 or more, at least 7 or more, at least 8 or more, at least 9 or more, at least 10 or more, at least 11 or more, at least 12 or more, at least 13 or more, at least 14 or more, at least 15 or more, at least 16 or more, at least 17 or more, at least 18 or more, at least 19 or more or at least 20 or more nucleotides with phosphorothioate linkages at the 3' end, *e.g.,* the 3' end of the exonuclease blocking oligonucleotide that does not bind to *(e.g.,* is not complementary to) the barcode sequence of the oligonucleotide. The exonuclease blocking oligonucleotide can include at least 10 or more modified nucleotides, *e.g.,* nucleotides with a phosphate backbone modification, *e.g.,* nucleotides with phosphorothioate linkages, at the 3' end. the modified nucleotides, *e.g.,* the nucleotides comprising the phosphorothioate linkage, are consecutive. The use of phosphorothioate linkages can prevent the DNA barcode from becoming double stranded (*e.g.,* upon contact with the exonuclease blocking oligonucleotide). The sample can be contacted with the exonuclease blocking oligonucleotide prior to performing the amplification process.

The sample can be contacted with the exonuclease blocking oligonucleotide for about 5 hours or less, about 4 hours or less, about 3 hours or less, about 2 hours or less, about 60 minutes or less, about 50 minutes or less, about 40 minutes or less or about 30 minutes or less. the sample can be contacted with the exonuclease blocking oligonucleotide for about 30 minutes.

One or more of the padlock oligonucleotides can hybridize to a blocking oligonucleotide (*e.g.,* an exonuclease blocking oligonucleotide) that is complementary to the oligonucleotide coupled to the protein binding reagent. Two or more, three or more, four or more, five or more, six or more, seven or more, eight or more, nine or more or ten or more padlock oligonucleotides can each bind to a distinct blocking oligonucleotide (*e.g.,* exonuclease blocking oligonucleotide). Each distinct blocking oligonucleotide (*e.g.,* exonuclease blocking oligonucleotide) hybridizes to an oligonucleotide coupled to a protein binding reagent.

One or more of a plurality of padlock oligonucleotides can hybridize to a blocking oligonucleotide (*e.g.,* exonuclease blocking oligonucleotide) that is complementary to the oligonucleotide coupled to a protein binding reagent and a second padlock oligonucleotide of the plurality of padlock oligonucleotides binds to a target nucleic acid, *e.g.,* an RNA or genomic nucleic acid of the sample.

The samples processed in accordance with the methods disclosed herein can be permeabilized for a second time, *e.g.,* prior to performing the amplification process, and after contacting the sample with the protein binding reagents. For example, such samples can be permeabilized the second time using a permeabilization reagent disclosed herein. permeabilization is performed using an acid. For example, but not by way of limitation, permeabilization can be performed using HCl, *e.g.,* from about 0.01N to about 10N HCl or from about 0.01N to about 1.0N HCl. permeabilization can be performed using a solution including an acid, *e.g.,* HCl, and a peptidase. The peptidase can be pepsin. The peptidase, *e.g.,* pepsin, can be included in the solution at a concentration from about 0.1 mg/ml to about 10 mg/ml or from about 0.1 mg/ml to about 5 mg/ml. Permeabilization can be performed using a solution including from about 0.01N to about 1N HCl and from about 0.1 mg/ml to about 5 mg/ml of a peptidase, e.g., pepsin. The sample can be contacted with HCl for about 1 to about 10 minutes.

The sample processed in accordance with the methods disclosed herein can be treated with NHS-acetate prior to the amplification process for nucleic acid detection. For example, but not by way of limitation, the sample can be treated with NHS-acetate following post-fixation and HCl permeabilization of the sample. The sample can be contacted with NHS-acetate for about 5 hours or less, about 4 hours or less, about 3 hours or less, about 2 hours or less, about 60 minutes or less, about 50 minutes or less, about 40 minutes or less or about 30 minutes or less. The sample can be contacted with NHS-acetate for about 30 minutes.

### D. Imaging Strategies

As described above, the methods of the present disclosure comprise imaging the target proteins and target nucleic acids in the sample. Oligonucleotides coupled to the protein binding reagents and/or the amplicons generated by an amplification process facilitate the imaging of the target proteins and nucleic acids.

A detection probe can be used to image the oligonucleotides bound to the protein binding reagents and/or the amplicons utilized in the methods disclosed herein. A "detection probe" refers to an oligonucleotide that can selectively hybridize to at least a portion of a target sequence (*e.g.,* a portion of a target sequence that has been amplified during RCA or portion of the oligonucleotide coupled to the protein binding reagent) under appropriate hybridization conditions. The detection probe can comprise or consist of about 10 to about 50 nucleotides, *e.g.,* about 15 to about 30 nucleotides. A detection probe for use in the present disclosure comprises a sequence that specifically hybridizes to an amplicon, *e.g.,* an RCA amplicon. A detection probe for use in the present disclosure comprises a sequence that specifically hybridizes to the DNA barcode sequence of the oligonucleotides coupled to the protein binding reagent, e.g., antibody. A detection probe for use in the present disclosure comprises a sequence that specifically hybridizes to a detection bridge oligonucleotide.

A detection bridge oligonucleotide for use in protein imaging is an oligonucleotide that hybridizes to a nucleotide sequence (*e.g.,* the DNA barcode sequence) of the oligonucleotides coupled to the protein binding reagent, *e.g.,* antibody. The detection bridge oligonucleotide can include a sequence that is complementary to the DNA barcode and additional nucleotides present in the oligonucleotide coupled to the protein binding reagent, *e.g.,* antibody, as shown in FIG. 1. For example, but not by way of limitation, the detection bridge oligonucleotide can include a sequence that is complementary to the DNA barcode and at least five additional nucleotides present in the oligonucleotide coupled to the protein binding reagent, *e.g.,* antibody. The detection bridge oligonucleotide can include a nucleotide sequence having a length of about 5 to about 100 nucleotides (*e.g.,* where the nucleotides are consecutive) that is complementary to the oligonucleotide coupled to the protein binding reagent, *e.g.,* antibody. Where the method includes the amplification of the exonuclease blocking oligonucleotide and/or the amplification of the oligonucleotide coupled to the protein binding reagent, the detection bridge oligonucleotide can include a sequence that is complementary to a nucleotide sequence of the resulting amplicon. The detection bridge oligonucleotide includes a nucleotide sequence having a length of about 5 to about 100 nucleotides (*e.g.,* where the nucleotides are consecutive) that is complementary to the nucleotide sequence of the resulting amplicon.

A detection bridge oligonucleotide for use in nucleic acid imaging is an oligonucleotide that hybridizes to a sequence that is present in the amplicons generated during the amplification reaction, *e.g.,* as shown in FIG. 1. The padlock oligonucleotides include such a sequence which is then amplified during an amplification process, *e.g.,* RCA. The amplicons include one or more, two or more, three or more, four or more or five or more sequences that can hybridize to a detection bridge oligonucleotide.

A detection bridge oligonucleotide can include two or more, three or more, four or more, five or more, six or more, seven or more, eight or more, nine or more or ten or more nucleotide sequences that can hybridize to a detection probe. A detection bridge oligonucleotide can include five or more nucleotide sequences that can hybridize to a detection probe. A detection bridge oligonucleotide can include ten or more nucleotide sequences that can hybridize to a detection probe. The detection bridge oligonucleotide can include from about one to about eight nucleotide sequences that can hybridize to a detection probe. The one or more nucleotide sequences that can hybridize to a detection probe can be identical.

The detection bridge oligonucleotide can be about 5 to about 200 nucleotides in length, e.g., about 5 to about 150 nucleotides in length, about 5 to about 100 nucleotides in length, about 5 to about 50 nucleotides in length, about 10 to about 150 nucleotides in length, about 20 to about 100 nucleotides in length or about 10 to about 100 nucleotides in length. The detection bridge oligonucleotide can be about 25 to about 100 nucleotides in length or about 25 to about 120 nucleotides in length. The detection bridge oligonucleotide can be about 25 to about 100 nucleotides in length. The detection bridge oligonucleotide can be about 25 to about 120 nucleotides in length. The detection bridge oligonucleotide can be one or more nucleotide sequences that can hybridize to a detection probe. The detection bridge oligonucleotide can be from about one to about eight nucleotide sequences that can hybridize to a detection probe. the detection bridge oligonucleotide can include from about one to about ten nucleotide sequences that can hybridize to a detection probe.

The detection bridge oligonucleotide can include a nucleotide sequence having a length of about 5 to about 100 nucleotides (*e.g.,* where the nucleotides are consecutive) that is complementary to a nucleotide sequence of an amplicon. The detection bridge oligonucleotide can include a nucleotide sequence having a length of about 5 to about 50 nucleotides (*e.g.,* where the nucleotides are consecutive) that is complementary to a nucleotide sequence of an amplicon. The detection bridge oligonucleotide can include a nucleotide sequence having a length of about 10 to about 40 nucleotides (*e.g.,* where the nucleotides are consecutive) that is complementary to a nucleotide sequence of an amplicon. The detection bridge oligonucleotide can include a nucleotide sequence having a length of about 10 to about 30 nucleotides (*e.g.,* where the nucleotides are consecutive) that is complementary to a nucleotide sequence of an amplicon. The detection bridge oligonucleotide can include a nucleotide sequence having a length of about 10 to about 20 nucleotides (*e.g.,* where the nucleotides are consecutive) that is complementary to a nucleotide sequence of an amplicon.

The detection bridge oligonucleotide for use in detecting a target nucleic acid (*e.g.,* an mRNA) can include a nucleotide sequence having a length of about 5 to about 40 nucleotides (*e.g.*, where the nucleotides are consecutive) that is complementary to a nucleotide sequence of an amplicon obtained from amplification of the target nucleic acid. The detection bridge oligonucleotide for use in detecting a target nucleic acid (*e.g.,* an mRNA) can include a nucleotide sequence having a length of about 5 to about 35 nucleotides (*e.g.*, where the nucleotides are consecutive) that is complementary to a nucleotide sequence of an amplicon obtained from amplification of the target nucleic acid. The detection bridge oligonucleotide for use in detecting a target nucleic acid (*e.g.,* an mRNA) can include a nucleotide sequence having a length of about 5 to about 30 nucleotides (*e.g.,* where the nucleotides are consecutive) that is complementary to a nucleotide sequence of an amplicon obtained from amplification of the target nucleic acid. The detection bridge oligonucleotide for use in detecting a target nucleic acid (*e.g.,* an mRNA) can include a nucleotide sequence having a length of about 5 to about 25 nucleotides (*e.g.,* where the nucleotides are consecutive) that is complementary to a nucleotide sequence of an amplicon obtained from amplification of the target nucleic acid. The detection bridge oligonucleotide for use in detecting a target nucleic acid (*e.g.,* an mRNA) can include a nucleotide sequence having a length of about 5 to about 20 nucleotides (*e.g.,* where the nucleotides are consecutive) that is complementary to a nucleotide sequence of an amplicon obtained from amplification of the target nucleic acid.

The detection bridge oligonucleotide for use in detecting an oligonucleotide coupled to a protein binding reagent can include a nucleotide sequence having a length of about 5 to about 50 nucleotides (*e.g.,* where the nucleotides are consecutive) that is complementary to the oligonucleotide coupled to a protein binding reagent. The detection bridge oligonucleotide for use in detecting an oligonucleotide coupled to a protein binding reagent can include a nucleotide sequence having a length of about 5 to about 40 nucleotides (*e.g.,* where the nucleotides are consecutive) that is complementary to the oligonucleotide coupled to a protein binding reagent. The detection bridge oligonucleotide for use in detecting an oligonucleotide coupled to a protein binding reagent can include a nucleotide sequence having a length of about 5 to about 35 nucleotides (*e.g.,* where the nucleotides are consecutive) that is complementary to the oligonucleotide coupled to a protein binding reagent. The detection bridge oligonucleotide for use in detecting an oligonucleotide coupled to a protein binding reagent can include a nucleotide sequence having a length of about 5 to about 30 nucleotides (*e.g.,* where the nucleotides are consecutive) that is complementary to the oligonucleotide coupled to a protein binding reagent. The detection bridge oligonucleotide for use in detecting an oligonucleotide coupled to a protein binding reagent can include a nucleotide sequence having a length of about 10 to about 30 nucleotides (*e.g.,* where the nucleotides are consecutive) that is complementary to the oligonucleotide coupled to a protein binding reagent. The detection bridge oligonucleotide for use in detecting an oligonucleotide coupled to a protein binding reagent can include a nucleotide sequence having a length of about 20 to about 30 nucleotides (*e.g.,* where the nucleotides are consecutive) that is complementary to the oligonucleotide coupled to a protein binding reagent.

The detection probe is conjugated to a detectable label to facilitate imaging. Non-limiting examples of detectable labels include fluorescent labels (such as fluorescein (*e.g., 5-*fluorescein, 6-carboxyfluorescein, 3'6-carboxyfluorescein, 5(6)-carboxyfluorescein, 6-hexachloro-fluorescein, 6-tetrachlorofluorescein, fluorescein isothiocyanate, and the like), rhodamine, phycobiliproteins and R-phycoerythrin and quantum dots (*e.g.,* zinc sulfide-capped cadmium selenide)), chromogenic labels, electron dense labels, chemiluminescent labels and radioactive labels. the detection probe is fluorescently labeled. the detection probe is covalently bound to a fluorescent label at its 5' end or 3' end.

The detection bridge oligonucleotide can include one or more, two or more, three or more, four or more, five or more, six or more, seven or more, eight or more, nine or more or ten or more detectable labels described herein, which can eliminate the need of a detection probe.

Where multiple target nucleic acids are to be imaged in a single sample, specific detection probes, *e.g.,* detection probes for specific target nucleic acids, can each be labeled with a different label, *e.g.,* fluorophore, thus allowing for simultaneous imaging of a plurality of target nucleic acids. Similarly, where multiple target nucleic acids are to be imaged in a single sample, specific detection probes, *e.g.,* detection probes for specific target nucleic acids, each can be labeled with a different label, *e.g.,* fluorophore, thus allowing for simultaneous imaging of a plurality of target nucleic acids.

Cyclic imaging is performed to visualize the target proteins and target nucleic acids labeled in a single sample. cyclic imaging involves the cyclic addition and removal of labeled detection probes. removal of labeled detection probes is achieved by thermal denaturing. Alternatively or additionally, removal of labeled detection probes is achieved by using detection probes with disulfide conjugated dyes thereby allowing the cleavage of the disulfide to remove the label from the detection probes.

Cyclic imaging of all the protein targets is performed first followed by cyclic imaging of all the nucleic acid targets. Alternatively, cyclic imaging of all the nucleic acid targets is performed first followed by cyclic imaging of all the protein targets. imaging of a first target protein is performed followed by the imaging of a first target nucleic acid and repeated until all protein and nucleic acid targets are imaged.

Cycling imaging can be used to detect target proteins in a sample. For example, but not by way of limitation, cyclic imaging can be performed by contacting the sample with a detection probe, *e.g.,* a fluorescently labeled detection probe, that is specific for the barcode sequence of the oligonucleotide conjugated to the antibody bound to the target sequence. the detection probe is then imaged and subsequently removed. The detection probe can be removed by a chaotropic solvent. One or more new detection probes are bound and subsequently imaged. This cyclic imaging process is repeated until all antibodies bound to target proteins in the sample have been imaged.

Cyclic imaging of the target nucleic acids can be performed. For example, but not by way of limitation, cyclic imaging can be performed by contacting the sample with a detection probe, *e.g.,* a fluorescently labeled detection probe, that is specific for the amplicons, *e.g.,* RCA amplicons, generated during the amplification of the target nucleic acid. The detection probe is then imaged and subsequently removed, *e.g.,* by a chaotropic solvent and/or by a thermal process. One or more new detection probes are bound and subsequently imaged. This cyclic imaging process is repeated until all target nucleic acids in the sample have been imaged.

Additional disclosure regarding cyclic imaging is provided in Black et al., Nature Protocols 16:3802-3835 (2021) and Kennedy-Darling et al., Eur. K. Immunol. 51(5):1262-1277 (2021).

A method for imaging a target protein and a target nucleic acid in a sample, can comprise: (a) providing a sample; (b) contacting the sample with a protein binding reagent that specifically binds to a target protein in the sample, wherein the protein binding reagent is coupled to an oligonucleotide; (c) contacting the sample with a blocking oligonucleotide (*e.g.,* exonuclease blocking oligonucleotide) comprising a nucleotide sequence that is complementary to a sequence of the oligonucleotide coupled to the protein binding reagent; (d) amplifying a target nucleic acid in the sample to generate an amplicon by performing an amplification process; (e) contacting the sample with a first labeled detection probe comprising a sequence that is complementary to a sequence of the oligonucleotide coupled to the protein binding reagent; (f) imaging the first labeled detection probe to detect the target protein; (g) contacting the sample with a second labeled detection probe comprising a sequence that is complementary to a sequence of the amplicon; and (h) imaging the second labeled detection probe to detect the target nucleic acid. The target nucleic acid is the blocking oligonucleotide (*e.g.,* the exonuclease blocking oligonucleotide) and/or the target nucleic acid is the oligonucleotide coupled to the protein binding reagent.

A method for imaging a target protein and a target nucleic acid in a sample, can comprise: (a) providing a sample; (b) contacting the sample with a protein binding reagent that specifically binds to a target protein in the sample, wherein the protein binding reagent is coupled to an oligonucleotide; (c) contacting the sample with a blocking oligonucleotide (*e.g.,* exonuclease blocking oligonucleotide) comprising a nucleotide sequence that is complementary to a sequence of the oligonucleotide coupled to the protein binding reagent; (d) amplifying a target nucleic acid in the sample to generate an amplicon by performing an amplification process; (e) contacting the sample with a first labeled detection probe comprising a sequence that is complementary to a sequence of the amplicon; (f) imaging the first labeled detection probe to detect the target nucleic acid; (g) contacting the sample with a second labeled detection probe comprising a sequence that is complementary to a sequence of the oligonucleotide coupled to the protein binding reagent; and (h) imaging the second labeled detection probe to detect the target protein. The target nucleic acid can be the blocking oligonucleotide and/or the target nucleic acid is the oligonucleotide coupled to the protein binding reagent.

A method for imaging a target protein and a target nucleic acid in a sample, can comprise: (a) providing a sample; (b) contacting the sample with a protein binding reagent that specifically binds to a target protein in the sample, wherein the protein binding reagent is coupled to an oligonucleotide; (c) contacting the sample with a blocking oligonucleotide (*e.g.,* exonuclease blocking oligonucleotide) comprising a nucleotide sequence that is complementary to a sequence of the oligonucleotide coupled to the protein binding reagent; (d) amplifying the blocking oligonucleotide and/or the oligonucleotide coupled to the protein binding reagent in the sample to generate a first amplicon by performing an amplification process; (e) amplifying a target nucleic acid in the sample to generate a second amplicon by performing the amplification process; (f) contacting the sample with a first labeled detection probe comprising a sequence that is complementary to a sequence of the oligonucleotide coupled to the protein binding reagent and/or contacting the sample with a first labeled detection probe comprising a sequence that is complementary to the first amplicon; (g) imaging the first labeled detection probe to detect the target protein; (h) contacting the sample with a second labeled detection probe comprising a sequence that is complementary to a sequence of the second amplicon; and (i) imaging the second labeled detection probe to detect the target nucleic acid. Generation of the first amplicon and the second amplicon occur simultaneously during a single amplification process. Generation of the first amplicon occurs during a first amplification process, and the second amplicon occur during a second amplification process, which are performed separately.

A method for imaging a target protein and a target nucleic acid in a sample, can comprise: (a) providing a sample; (b) contacting the sample with a protein binding reagent that specifically binds to a target protein in the sample, wherein the protein binding reagent is coupled to an oligonucleotide; (c) contacting the sample with a blocking oligonucleotide, e.g., an exonuclease blocking oligonucleotide, comprising a nucleotide sequence that is complementary to a sequence of the oligonucleotide coupled to the protein binding reagent; (d) amplifying the blocking oligonucleotide and/or the oligonucleotide coupled to the protein binding reagent to generate a first amplicon by performing an amplification process; (e) amplifying a target nucleic acid in the sample to generate a second amplicon by performing the amplification process; (f) contacting the sample with a first labeled detection probe comprising a sequence that is complementary to a sequence of the second amplicon; (g) imaging the first labeled detection probe to detect the target nucleic acid; (h) contacting the sample with a second labeled detection probe comprising a sequence that is complementary to a sequence of the oligonucleotide coupled to the protein binding reagent and/or contacting the sample with a second labeled detection probe comprising a sequence that is complementary to the first amplicon; and (i) imaging the second labeled detection probe to detect the target protein. Generation of the first amplicon and the second amplicon occur simultaneously during a single amplification process. Generation of the first amplicon occurs during a first amplification process, and the second amplicon occur during a second amplification process, which are performed separately.

A method for imaging a target protein and a target nucleic acid in a sample, can comprise: (a) binding a protein binding reagent coupled to an oligonucleotide to a target protein in the sample; (b) hybridizing a blocking oligonucleotide, *e.g.,* an exonuclease blocking oligonucleotide, to the oligonucleotide coupled to the protein binding reagent; (c) amplifying a target nucleic acid in the sample to generate an amplicon by performing an amplification process; (d) imaging the target protein by detecting a first labeled detection probe that is hybridized to the oligonucleotide coupled to the protein binding reagent; and (e) imaging the target nucleic acid by detecting a second labeled detection probe that is hybridized to the amplicon. The target nucleic acid can be the blocking oligonucleotide and/or the target nucleic acid is the oligonucleotide coupled to the protein binding reagent.

A method for imaging a target protein and a target nucleic acid in a sample, can comprise: (a) binding a protein binding reagent coupled to an oligonucleotide to a target protein in the sample; (b) hybridizing a blocking oligonucleotide to the oligonucleotide coupled to the protein binding reagent; (c) amplifying a target nucleic acid in the sample to generate an amplicon by performing an amplification process; (d) imaging the target nucleic acid by detecting a first labeled detection probe that is hybridized to the amplicon; and (e) imaging the target protein by detecting a second labeled detection probe that is hybridized to the oligonucleotide coupled to the protein binding reagent. The target nucleic acid can be the blocking oligonucleotide and/or the target nucleic acid is the oligonucleotide coupled to the protein binding reagent.

A method for imaging a target protein and a target nucleic acid in a sample, can comprise: (a) binding a protein binding reagent coupled to an oligonucleotide to a target protein in the sample; (b) hybridizing a blocking oligonucleotide, *e.g.,* an exonuclease blocking oligonucleotide, to the oligonucleotide coupled to the protein binding reagent; (c) amplifying the blocking oligonucleotide and/or the oligonucleotide coupled to the protein binding reagent in the sample to generate a first amplicon by performing an amplification process; (d) amplifying a target nucleic acid in the sample to generate a second amplicon by performing the amplification process; (e) imaging the target protein by detecting a first labeled detection probe that is hybridized to the oligonucleotide coupled to the protein binding reagent and/or hybridized to the first amplicon; and (f) imaging the target nucleic acid by detecting a second labeled detection probe that is hybridized to the second amplicon. Generation of the first amplicon and the second amplicon occur simultaneously during a single amplification process. Generation of the first amplicon can occur during a first amplification process and the second amplicon occur during a second amplification process, which are performed separately.

A method for imaging a target protein and a target nucleic acid in a sample can include (a) providing a sample, (b) contacting the sample with a protein binding reagent that specifically binds to a target protein in the sample, wherein the protein binding reagent is coupled to an oligonucleotide, (c) contacting the sample with a blocking oligonucleotide, e.g., an exonuclease blocking oligonucleotide, comprising a nucleotide sequence that is complementary to a sequence of the oligonucleotide coupled to the protein binding reagent, (d) amplifying a target nucleic acid in the sample to generate an amplicon by performing an amplification process, (e) contacting the sample with a first bridging oligonucleotide comprising a sequence that is complementary to a sequence of the oligonucleotide coupled to the protein binding reagent and contacting the sample with a first labeled detection probe comprising a sequence that is complementary to a sequence of the first bridging oligonucleotide, (f) imaging the first labeled detection probe to detect the target protein, (g) contacting the sample with a second bridging oligonucleotide comprising a sequence that is complementary to a sequence of the amplicon and contacting the sample with a second labeled detection probe comprising a sequence that is complementary to a sequence of the second bridging oligonucleotide and (h) imaging the second labeled detection probe to detect the target nucleic acid.

A method for imaging a target protein and a target nucleic acid in a sample can include (a) providing a sample, (b) contacting the sample with a protein binding reagent that specifically binds to a target protein in the sample, wherein the protein binding reagent is coupled to an oligonucleotide, (c) contacting the sample with a blocking oligonucleotide, e.g., an exonuclease blocking oligonucleotide, comprising a nucleotide sequence that is complementary to a sequence of the oligonucleotide coupled to the protein binding reagent, (d) amplifying a target nucleic acid in the sample to generate an amplicon by performing an amplification process, (e) contacting the sample with a first bridging oligonucleotide comprising a sequence that is complementary to a sequence of the amplicon and contacting the sample with a first labeled detection probe comprising a sequence that the amplicon, (f) imaging the first labeled detection probe to detect the target nucleic acid, (g) contacting the sample with a second bridging oligonucleotide comprising a sequence that is complementary to a sequence of the oligonucleotide coupled to the protein binding reagent and contacting the sample with a second labeled detection probe comprising a sequence that is complementary to a sequence of the second bridging oligonucleotide and (h) imaging the second labeled detection probe to detect the target protein.

### III. SYSTEMS AND KITS

The present disclosure describes systems and kits for performing the methods of the present disclosure. For example, but not by way of limitation, the present disclosure provides systems and kits containing materials for performing a method for imaging of a nucleic acid and a protein in a sample.

A system or kit of the present disclosure can include a container containing one or more protein binding reagents. The system or kit can further include a container containing one or more blocking oligonucleotides. A system or kit of the present disclosure can further include one or more detection probes, *e.g.,* fluorescently labeled detection probes. Non-limiting examples of suitable containers include bottles, test tubes, vials and microtiter plates. The containers can be formed from a variety of materials such as glass or plastic.

The system or kit can further include a package insert that provides instructions for using the components provided in the system or kit. For example, a system or kit of the present disclosure can include a package insert that provides instructions for performing methods for imaging one or more target proteins and one or more target nucleic acids in a single sample.

A system or kit of the present disclosure can further include reagents for performing an amplification reaction, *e.g.,* an RCA reaction. The reagents can include one more of the following: polymerases, reverse transcriptases, nucleoside triphosphates or NTP analogues, primers, cofactors, ligation reaction reagents, endonucleases, lysis reagents, dyes, markers, RNase inhibitors and labels.

The system or kit can include other materials desirable from a commercial and user standpoint, including other buffers and diluents. The system or kit can include materials or reagents for permeabilizing, fixing and/or crosslinking the cells or nuclei. A system or kit of the present disclosure can include a fixative, *e.g.,* formaldehyde. A system or kit can include NHS-Acetate. A system or kit can include HCl.

The components of the system or kit are provided in predetermined ratios, with the relative amounts of the various reagents suitably varied to obtain the desired sensitivity and throughput of the disclosed methods.

### EXAMPLES

The presently disclosed subject matter will be better understood by reference to the following example, which is provided as exemplary of the presently disclosed subject matter, and not by way of limitation.

### Example 1: Simultaneous imaging of RNA and protein in a sample

This example describes a method for detecting RNA and protein in a single biological sample, as shown in FIG. 1.

### Preparing Sample:

The method begins by preparing the sample, e.g., a section of sample, for protein and nucleic acid detection. If the sample is frozen, the sample can be thawed from -80°C for 5 minutes in a covered chamber. Preparing the sample begins by fixing the sample in 4% Formaldehyde in 1X PBS for 5 minutes to 1 hour. The sample was dehydrated with an ethanol series, *i.e.,* 70%, 85% and 100%, for 1 minute each. The sample was then dried at RT in a closed container for 5 minutes or until dry. The sample was mounted to a flow cell and washed 2 times with PBS-Tween (PBST).

### Antibody Staining:

The sample was subsequently stained for a protein by using an antibody specific for the protein and coupled to an oligonucleotide, where the oligonucleotide includes a barcode sequence. The sample is permeabilized for 20 minutes with PBS + TritonX (0.5%) + 1:80 RiboLock. The sample was blocked for 15 minutes to overnight at 4°C or room temperature and stained with one or more Total-Seq antibodies for 1.5 hours to overnight at 4°C or room temperature. The blocking solution further includes oligonucleotides that are complementary to conserved sequences in the oligonucleotide bound to the antibody. It was found that making RNA accessible in many tissue types required treatment with hydrochloric acid (HCl) and often a proteinase (as noted below), and that such treatment makes RNA and protein detection in a single sample by imaging difficult. In particular, the HCl treatment for permeabilizing a sample for RNA detection abolished antibody binding. Therefore, to retain antibody attachment during RNA staining, the sample was subsequently washed 2 times with PBST and post-fixed in 4% PFA or in 4% PFA and 1% glutaraldehyde for 5 minutes to 1 hour and washed 3 times with PBST. This post-fixing treatment retained antibodies while facilitating RNA hybridization even in HCl denatured tissue.

### mRNA Detection:

Following protein staining, the sample was stained for mRNA by the following protocol. The sample that was stained for antibodies, as described above, was permeabilized in 0.1N HCl for 3 minutes. The solution containing 0.1N HCl can also include 1 mg/ml of pepsin. HCl permeabilization was performed after antibody staining because it was found that treatment with HCl before antibody staining significantly decreased and modified antibody staining patterns. It was also found that RNA staining in tissue was suboptimal after the preprocessing steps performed for protein and RNA staining. To facilitate improved staining, samples were treated with NHS-acetate prior to RNA hybridization but after HCl permeabilization. In particular, the sample was washed 5 times with PBST and treated with NHS-Acetate for 30 minutes.

The sample was then blocked for 30 minutes in hybridization blocking buffer and Phi29 blocking oligonucleotides and washed 2 times in PBST. The Phi29 blocking oligonucleotides can have the structure shown in FIG. 4. The Phi29 blocking oligonucleotides were used to protect the oligonucleotides conjugated to the antibodies from the Phi29 3'→5' exonuclease activity, which can rapidly degrade the single stranded 3' end of the oligonucleotides, as shown in the right panel of FIG. 4. The Phi29 blocking oligonucleotides have two features: (1) a long 5' extension template, which was found to prevent the exonuclease activity of Phi29 on the antibody-oligonucleotide conjugate and (2) a 3' flap with at least 10 phosphorothioate linkages, which was found to prevent Phi29 from making the DNA barcode double stranded and maintain hybridization readouts of the DNA barcode. The 3' flap of the Phi29 blocking oligonucleotides can have a length of about 10-20 nucleotides with at least 10 phosphorothioate linkages. The 5' extension template of the Phi29 blocking oligonucleotides can have a length of about 15-25 nucleotides such as about 22 nucleotides. The length of the nucleotide sequence present in the Phi29 blocking oligonucleotides that is complementary to the oligonucleotide of the antibody-oligonucleotide conjugate can be about 20-30 nucleotides. As shown in Fig. 4, the use of Phi29 blocking oligonucleotides greatly improved the sensitivity of protein detection.

The samples were then hybridized at 45°C in 20% formamide hybridization buffer and 10 nM of padlock oligonucleotides for 16-18 hours. The sample was washed 3 times with PBST. Ligation was performed overnight with SPLINTR ligase at RT and subsequently washed 3 times with PBST. Rolling circular amplification (RCA) was performed overnight at 30°C. The sample was washed 2 times with PBST and post-fixed for 15 minutes in 4% PFA. During RCA, the polymerase activity of the Phi29 polymerase extends the 3' end of the oligonucleotide conjugated to the antibody to generate a complementary sequence to the 5' extension template of the Phi29 blocking oligonucleotide, as shown in FIG. 4.

### Cyclic Imaging:

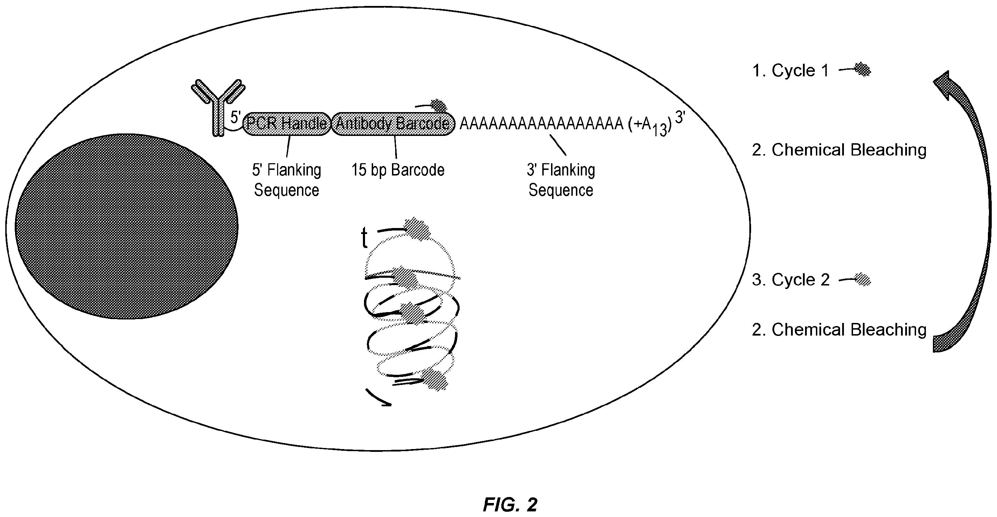

Imaging of the target proteins and target nucleic acids were performed by cyclic imaging as shown in FIG. 2, FIG. 3 and FIG. 5.

For protein detection, the protein detection bridge oligonucleotides and accompanying readout oligonucleotides were hybridized to the oligonucleotides coupled to the antibodies as shown in FIG. 1. The protein detection bridge oligonucleotides bind to the barcode sequence on the antibody-oligonucleotide conjugate and 5 nucleotides of a conserved sequence on the oligonucleotide to maximize stability. The protein detection bridge oligonucleotides have a nucleotide sequence of about 28 nucleotides in length that is complementary to the oligonucleotide of the antibody-oligonucleotide conjugate. The protein detection bridge oligonucleotides include repeats of a nucleotide sequence that bind to fluorescently labeled probes to amplify the fluorescent signal detected. The samples were then washed and imaged. This cycle was repeated until all proteins of interest were imaged. To remove probes and protein detection bridge oligonucleotides prior to the next imaging cycle, thermal denaturation was performed. Alternatively, the use of probes with disulfide conjugated dyes were used which allows the cleavage of the disulfide to remove the fluorescent signal from the probes.

For mRNA detection, the mRNA detection bridge oligonucleotides and accompanying readout oligonucleotides were hybridized to mRNAs of interest. The mRNA detection bridge oligonucleotides include nucleotide sequences that are complementary to the amplicons generated during RCA (*e.g.,* sequences present in the padlock oligonucleotides) and also include repeats of a nucleotide sequence that bind to fluorescently labeled probes to amplify the fluorescent signal detected. The mRNA detection bridge oligonucleotides have a nucleotide sequence of about 12 nucleotides in length that is complementary to the amplicon. The samples were then washed and imaged. This cycle was repeated until all mRNAs of interest were imaged. As noted above for protein detection, thermal denaturation or probes with disulfide conjugated dyes were used to allow cyclic imaging.

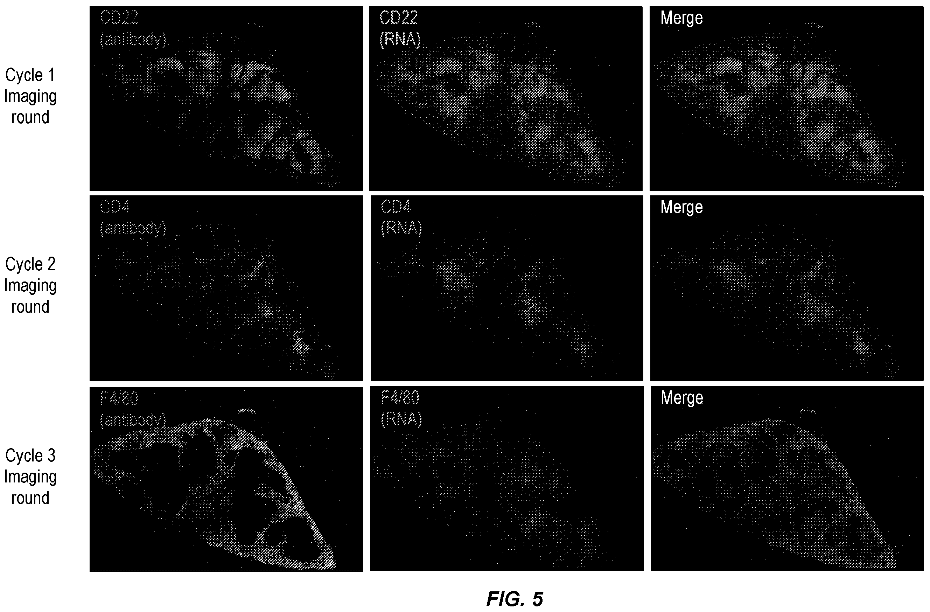

As shown in FIG. 3 and FIG. 5, this method allows the simultaneous detection of multiple proteins and an mRNA in a single sample

## Claims

1. A method for imaging a target protein and a target nucleic acid in a sample, comprising:
(a) using a provided sample;
(b) contacting the sample with a protein binding reagent that specifically binds to a target protein in the sample, wherein the protein binding reagent is coupled to an oligonucleotide;
(c) contacting the sample with a blocking oligonucleotide comprising a nucleotide sequence that is complementary to a sequence of the oligonucleotide coupled to the protein binding reagent;
(d) amplifying a target nucleic acid in the sample to generate an amplicon by performing an amplification process;
(e) contacting the sample with a first labeled detection probe comprising a sequence that is complementary to a sequence of the oligonucleotide coupled to the protein binding reagent;
(f) imaging the first labeled detection probe to detect the target protein;
(g) contacting the sample with a second labeled detection probe comprising a sequence that is complementary to a sequence of the amplicon; and
(h) imaging the second labeled detection probe to detect the target nucleic acid.

2. A method for imaging a target protein and a target nucleic acid in a sample, comprising:
(a) binding a protein binding reagent coupled to an oligonucleotide to a target protein in the sample;
(b) hybridizing a blocking oligonucleotide to the oligonucleotide coupled to the protein binding reagent;
(c) amplifying a target nucleic acid in the sample to generate an amplicon by performing an amplification process;
(d) imaging the target protein by detecting a first labeled detection probe that is hybridized to the oligonucleotide coupled to the protein binding reagent; and
(e) imaging the target nucleic acid by detecting a second labeled detection probe that is hybridized to the amplicon.

3. The method of claim 1 or 2, wherein the amplification process is a rolling circle amplification process.

4. The method of claim 3, wherein the rolling circle amplification process comprises:
(a) contacting the sample with (i) a padlock probe comprising two nucleotide sequences that are complementary to the target nucleic acid and (ii) a ligase to generate a circular DNA template; and
(b) performing a rolling circle amplification process to generate an amplicon from the circular DNA template.

5. The method of any one of claims 1-4, wherein the protein binding reagent is an antibody or an antigen binding fragment thereof.

6. The method of any one of claims 1-5, wherein the target nucleic acid comprises RNA.

7. The method of any one of claims 1-5, wherein the target nucleic acid is the oligonucleotide coupled to the protein binding reagent, the blocking oligonucleotide or a combination thereof.

8. The method of any one of claims 1-7, wherein the nucleotide sequence that is complementary to the oligonucleotide coupled to protein binding reagent is located at the 5' end of the blocking oligonucleotide.

9. The method of any one of claims 1-8, wherein the oligonucleotide coupled to protein binding reagent comprises a barcode sequence, and wherein the blocking oligonucleotide does not bind to the barcode sequence.

10. The method of any one of claims 1-9, wherein the blocking oligonucleotide comprises one or more modified nucleotides.

11. The method of claim 10, wherein the 3' end of the blocking oligonucleotide comprises from about 1 to about 10 nucleotides with a phosphorothioate linkage.

12. The method of any one of claims 1-11, wherein providing a sample comprises one or more of the following:
(a) treating the sample with a fixative;
(b) dehydrating the sample; and
(c) permeabilizing the sample.

13. The method of any one of claims 1-12, wherein at least 10 target proteins and at least 10 target nucleic acids are imaged in the sample.

14. The method of any one of claims 1-13, wherein the sample is treated with NHS-acetate prior to amplifying the target nucleic acid.

15. The method of any one of claims 1-14, wherein the sample is a tissue sample.

## Patentansprüche

1. Verfahren zur Abbildung eines Zielproteins und einer Zielnukleinsäure in einer Probe, umfassend:
(a) Verwenden einer bereitgestellten Probe;
(b) Inkontaktbringen der Probe mit einem Proteinbindungsreagens, das spezifisch an ein Zielprotein in der Probe bindet, wobei das Proteinbindungsreagens an ein Oligonukleotid gekoppelt ist;
(c) Inkontaktbringen der Probe mit einem blockierenden Oligonukleotid, umfassend eine Nukleotidsequenz, die komplementär zu einer Sequenz des Oligonukleotids ist, das an das Proteinbindungsreagens gekoppelt ist;
(d) Amplifizieren einer Zielnukleinsäure in der Probe zur Erzeugung eines Amplikon mittels Durchführen eines Amplifikationsverfahrens;
(e) Inkontaktbringen der Probe mit einer ersten markierten Nachweissonde, umfassend eine Sequenz, die komplementär zu einer Sequenz des Oligonukleotids ist, das an das Proteinbindungsreagens gekoppelt ist;
(f) Abbilden der ersten markierten Nachweissonde zum Nachweis des Zielproteins;
(g) Inkontaktbringen der Probe mit einer zweiten markierten Nachweissonde, umfassend eine Sequenz, die komplementär zu einer Sequenz des Amplikon ist; und
(h) Abbildung der zweiten markierten Nachweissonde zum Nachweis der Zielnukleinsäure.

2. Verfahren zur Abbildung eines Zielproteins und einer Zielnukleinsäure in einer Probe, umfassend:
(a) Binden eines Proteinbindungsreagens, das an ein Oligonukleotid gekoppelt ist, an ein Zielprotein in der Probe;
(b) Hybridisieren eines blockierenden Oligonukleotids an das Oligonukleotid, das an das Proteinbindungsreagens gekoppelt ist;
(c) Amplifizieren einer Zielnukleinsäure in der Probe zur Erzeugung eines Amplikon mittels Durchführen eines Amplifikationsverfahrens;
(d) Abbilden des Zielproteins durch Nachweis einer ersten markierten Nachweissonde, die an das Oligonukleotid hybridisiert ist, das an das Proteinbindungsreagens gekoppelt ist; und
(e) Abbilden der Zielnukleinsäure durch Nachweis einer zweiten markierten Nachweissonde, die an das Amplikon hybridisiert ist.

3. Verfahren nach Anspruch 1 oder 2, wobei das Amplifikationsverfahren ein Rolling-Circle-Amplifikationsverfahren ist.

4. Verfahren nach Anspruch 3, wobei das Rolling-Circle-Amplifikationsverfahren Folgendes umfasst:
(a) Inkontaktbringen der Probe mit (i) einer Padlock-Sonde, umfassend zwei Nukleotidsequenzen, die komplementär zu der Zielnukleinsäure sind, und (ii) einer Ligase zur Erzeugung einer zirkulären DNA-Matrize; und
(b) Durchführen eines Rolling-Circle-Amplifikationsverfahrens zur Erzeugung eines Amplikon aus der zirkulären DNA-Matrize.

5. Verfahren nach einem der Ansprüche 1-4, wobei das Proteinbindungsreagens ein Antikörper oder ein antigenbindendes Fragment davon ist.

6. Verfahren nach einem der Ansprüche 1-5, wobei die Zielnukleinsäure RNA umfasst.

7. Verfahren nach einem der Ansprüche 1-5, wobei die Zielnukleinsäure das Oligonukleotid, das an das Proteinbindungsreagens gekoppelt ist, das blockierende Oligonukleotid oder eine Kombination davon ist.

8. Verfahren nach einem der Ansprüche 1-7, wobei sich die Nukleotidsequenz, die komplementär zu dem Oligonukleotid ist, das an das Proteinbindungsreagens gekoppelt ist, am 5'-Ende des blockierenden Oligonukleotids befindet.

9. Verfahren nach einem der Ansprüche 1-8, wobei das Oligonukleotid, das an das Proteinbindungsreagens gekoppelt ist, eine Barcode-Sequenz umfasst und wobei das blockierende Oligonukleotid nicht an die Barcode-Sequenz bindet.

10. Verfahren nach einem der Ansprüche 1-9, wobei das blockierende Oligonukleotid ein oder mehrere modifizierte Nukleotide umfasst.

11. Verfahren nach Anspruch 10, wobei das 3'-Ende des blockierenden Oligonukleotids etwa 1 bis etwa 10 Nukleotide mit einer Phosphorthioat-Verknüpfung umfasst.

12. Verfahren nach einem der Ansprüche 1-11, wobei das Bereitstellen einer Probe eines oder mehrere der Folgenden umfasst:
(a) Behandeln der Probe mit einem Fixiermittel;
(b) Dehydrieren der Probe; und
(c) Permeabilisieren der Probe.

13. Verfahren nach einem der Ansprüche 1-12, wobei mindestens 10 Zielproteine und mindestens 10 Zielnukleinsäuren in der Probe abgebildet werden.

14. Verfahren nach einem der Ansprüche 1-13, wobei die Probe vor dem Amplifizieren der Zielnukleinsäure mit NHS-Acetat behandelt wird.

15. Verfahren nach einem der Ansprüche 1-14, wobei die Probe eine Gewebeprobe ist.

## Revendications

1. Procédé d'imagerie d'une protéine cible et d'un acide nucléique cible dans un échantillon, comprenant :
(a) l'utilisation d'un échantillon fourni ;
(b) la mise en contact de l'échantillon avec un réactif se liant à une protéine qui se lie spécifiquement à une protéine cible dans l'échantillon, le réactif se liant à une protéine étant couplé à un oligonucléotide ;
(c) la mise en contact de l'échantillon avec un oligonucléotide bloquant comprenant une séquence nucléotidique qui est complémentaire d'une séquence de l'oligonucléotide couplé au réactif se liant à une protéine ;
(d) l'amplification d'un acide nucléique cible dans l'échantillon pour générer un amplicon en réalisant un processus d'amplification ;
(e) la mise en contact de l'échantillon avec une première sonde de détection marquée comprenant une séquence qui est complémentaire d'une séquence de l'oligonucléotide couplé au réactif se liant à une protéine ;
(f) l'imagerie de la première sonde de détection marquée pour détecter la protéine cible ;
(g) la mise en contact de l'échantillon avec une seconde sonde de détection marquée comprenant une séquence qui est complémentaire d'une séquence de l'amplicon ; et
(h) l'imagerie de la seconde sonde de détection marquée pour détecter l'acide nucléique cible.

2. Procédé d'imagerie d'une protéine cible et d'un acide nucléique cible dans un échantillon, comprenant :
(a) la liaison d'un réactif se liant à une protéine couplé à un oligonucléotide à une protéine cible dans l'échantillon ;
(b) l'hybridation d'un oligonucléotide bloquant à l'oligonucléotide couplé au réactif se liant à une protéine ;
(c) l'amplification d'un acide nucléique cible dans l'échantillon pour générer un amplicon en réalisant un processus d'amplification ;
(d) l'imagerie de la protéine cible en détectant une première sonde de détection marquée qui est hybridée à l'oligonucléotide couplé au réactif se liant à une protéine ; et
(e) l'imagerie de l'acide nucléique cible en détectant une seconde sonde de détection marquée qui est hybridée à l'amplicon.

3. Procédé selon la revendication 1 ou 2, dans lequel le processus d'amplification est un processus d'amplification en cercle roulant.

4. Procédé selon la revendication 3, dans lequel le processus d'amplification en cercle roulant comprend :
(a) la mise en contact de l'échantillon avec (i) une sonde cadenas comprenant deux séquences nucléotidiques qui sont complémentaires de l'acide nucléique cible et (ii) une ligase pour générer une matrice d'ADN circulaire ; et
(b) la réalisation d'un processus d'amplification en cercle roulant pour générer un amplicon à partir de la matrice d'ADN circulaire.

5. Procédé selon l'une quelconque des revendications 1 à 4, dans lequel le réactif se liant à une protéine est un anticorps ou un fragment se liant à l'antigène de celui-ci.

6. Procédé selon l'une quelconque des revendications 1 à 5, dans lequel l'acide nucléique cible comprend l'ARN.

7. Procédé selon l'une quelconque des revendications 1 à 5, dans lequel l'acide nucléique cible est l'oligonucléotide couplé au réactif se liant à une protéine, l'oligonucléotide bloquant ou une combinaison de ceux-ci.

8. Procédé selon l'une quelconque des revendications 1 à 7, dans lequel la séquence nucléotidique qui est complémentaire de l'oligonucléotide couplé au réactif se liant à une protéine est située au niveau de l'extrémité 5' de l'oligonucléotide bloquant.

9. Procédé selon l'une quelconque des revendications 1 à 8, dans lequel l'oligonucléotide couplé au réactif se liant à une protéine comprend une séquence de code-barres, et dans lequel l'oligonucléotide bloquant ne se lie pas à la séquence de code-barres.

10. Procédé selon l'une quelconque des revendications 1 à 9, dans lequel l'oligonucléotide bloquant comprend un ou plusieurs nucléotides modifiés.

11. Procédé selon la revendication 10, dans lequel l'extrémité 3' de l'oligonucléotide bloquant comprend d'environ 1 à environ 10 nucléotides avec une liaison phosphorothioate.

12. Procédé selon l'une quelconque des revendications 1 à 11, dans lequel la fourniture d'un échantillon comprend un ou plusieurs de ce qui suit :
(a) traitement de l'échantillon avec un fixateur ;
(b) déshydratation de l'échantillon ; et
(c) perméabilisation de l'échantillon.

13. Procédé selon l'une quelconque des revendications 1 à 12, dans lequel au moins 10 protéines cibles et au moins 10 acides nucléiques cibles sont imagés dans l'échantillon.

14. Procédé selon l'une quelconque des revendications 1 à 13, dans lequel l'échantillon est traité avec du NHS-acétate avant l'amplification de l'acide nucléique cible.

15. Procédé selon l'une quelconque des revendications 1 à 14, dans lequel l'échantillon est un échantillon de tissu.
